# FASCICULE DE BREVET EUROPEEN

(11) **EP 0 315 519 B1**
(45) Date de publication et mention de la délivrance du brevet: **24.03.1993**
(21) Numéro de dépôt: 88402741.8
(22) Date de dépôt: 02.11.1988
(51) Int. Cl.: C07C 229/30, C07K 5/06, C07C 237/02, A61K 31/16, A61K 31/195

(54) **Nouveaux dérivés de l'acide aminopimélique, leur procédé de préparation et leur application comme médicaments**
Aminopimelinsäure-Derivate, Verfahren zu deren Herstellung und deren Verwendung als Arzneimittel
Aminopimelic acid derivatives, process for their preparation and their use as medicines

(30) Priorité: 03.11.1987 FR 8715209
(43) Date de publication de la demande: 10.05.1989
(73) Titulaire: ROUSSEL-UCLAF, 75007 Paris (FR)
(72) Inventeur: Agouridas, Constantin, F-75007 Paris (FR); Fauveau, Patrick, F-93190 Livry-Gargan (FR); Damais, Chantal, F-75015 Paris (FR)
(74) Mandataire: Tonnellier, Marie-José

(56) Documents cités:
- FR-A- 2 566 410
- CHEMICAL ABSTRACTS, vol. 100, no. 19, 7 mai 1984, page 299, résumé no. 153809d, Columbus, Ohio, US; N. MURAKAMI et al.: "D-2-Aminopimelic acid and trans-3,4-dehydro-D-2-aminopimelic acid from Asplenium unilaterale"
- CHEMICAL ABSTRACTS, vol. 83, no. 21, 24 novembre 1975, page 295, résumé no. 175488f, Columbus, Ohio, US; S. HATANAKA et al.: "Biochemical studies on nitrogen compounds of fungi. XII. l-2-Amino-4-methylpimelic acid, a new amino acid from Lactarius species"
- JOURNAL OF MEDICINAL CHEMISTRY, vol. 29, no. 1, janvier 1986, pages 89-95, American Chemical Society, Washington, DC, US; D.A. BERGES et al.: "Peptides of 2-aminopimelic acid: antibacterial agents that inhibit diaminopimelic acid biosynthesis"
- JOURNAL OF MEDICINAL CHEMISTRY, vol. 30, no. 6, juin 1987, pages 1074-1090, American Chemical Society, Washington, DC, US; D.W. GRAHAM et al.: "Inhibition of the mammalian beta-lactamase renal dipeptidase (dehydropeptidase-I) by (Z)-2-(acylamino)-3-substituted-propenoic acids"
- SYNTHESIS, mars 1988, pages 173-175, Stuttgart, DE; D. PETTIG et al.: "Asymmetric synthesis via heterocyclic intermediates; XXXVII. Asymmetric synthesis of dimethyl (R)-2-amino-(E)-hept-4-enedioates by the bislactim ether method"

## Description

La présente invention concerne des dérivés de l'acide aminopimélique, leur procédé de préparation et leur application code médicaments.

On connaissait déjà des dérivés de l'acide aminopimélique et de l'acide diaminopimélique présentant des propriétés antibiotiques (cf CHEMICAL ABSTRACTS Vol 100 n^{o} 19, 7 Mai 1984, page 299, résumé 153809 d, CHEMICAL ABSTRACTS Vol 84 n^{o} 21, 24 Novembre 1975, page 295, résumé 175488 f, FR A 2566410, JOURNAL OF MEDICINAL CHEMISTRY Vol 29 n^{o} 1, Janvier 1986, pages 89-95, JOURNAL OF MEDICINAL CHEMISTRY Vol 30 n^{o} 6, Juin 1987, pages 1074-1090). On vient de découvrir de nouveaux dérivés de l'acide aminopimélique présentant des propriétés antibiotiques et immunomodulatrices.

L'invention a pour objet les composés de formule (I) : 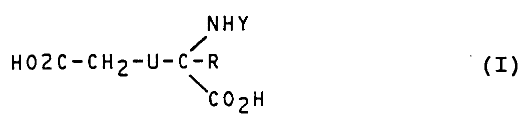
dans laquelle U représente un radical 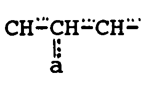
dans lequel les traits pointillés représentent une seule double liaison de configuration cis ou trans et a représente un atome d'hydrogène, un radical méthyle ou un groupement méthylène -CH₂-, Y représente un atome d'hydrogène ou le reste d'un acide aminé lié par le carboxyle alpha ou oméga ou d'un peptide constitué de 2, 3 ou 4 acides aminés dont l'amine est éventuellement acylé par un acide aliphatique saturé ou insaturé renfermant de 6 à 24 atomes de carbone et R représente un atome d'hydrogène ou un radical alkyle, linéaire ou ramifié, saturé ou insaturé, renfermant jusqu'à 8 atomes de carbone éventuellement substitué par un ou plusieurs atomes d'halogène, à l'exception de l'acide trans 3,4-déhydro-D-2-aminopimélique, ainsi que leurs esters avec les alcools renfermant jusqu'à 6 atomes de carbone, leurs amides primaires et secondaires et leurs sels avec les acides organiques ou minéraux ou avec les bases.

U représente un radical :

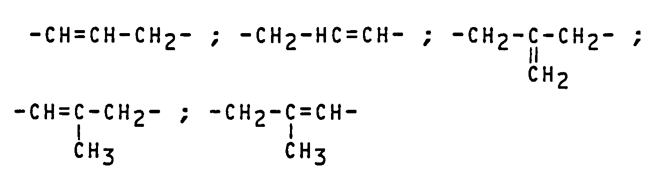

Lorsque R représente un radical alkyle, il s'agit de préférence du radical méthyle, éthyle, propyle, isopropyle ou butyle ou d'un radical insaturé comme le radical vinyle, allyle, éthynyle ou propynyle.

Lorsque R représente un radical alkyle substitué par un ou plusieurs atomes d'halogène, il s'agit de préférence d'un radical alkyle substitué par un ou plusieurs atomes de fluor ou de chlore, par exemple, les radicaux -CHF₂, -CH₂F, -CHCl₂, -CH₂Cl.

Dans la formule générale I et dans ce qui suit, l'acide aminé est de préférence un acide alpha-aminé et peut être choisi dans le groupe constitué par Ala, Val, Ival, Leu, Ile, Asp, Asn, Glu, Gln, Ser, Thr, Cys, Met Lys, Arg, Phe, Tyr, Trp, His et Pro, Nva, Nle, Hyp, Orn, ces acides étant sous la forme D ou L ainsi que par Sar et Gly, tous les acides précédemment nommés pouvant être N acylés ou N-alkylés dans le cas d'un peptide comprenant 2, 3 ou 4 acides aminés, ceux-ci sont choisis dans le groupe constitué par les acides aminés ci-dessus.

On admettra par convention que les symboles des acides alpha-amino carboxyliques représentent ces acides sous leur configuration D ou L (par exemple, le terme Ala signifie Alanine sous forme D ou sous forme L).

L'acide aliphatique saturé ou insaturé renferme de 6 à 24 atomes de carbone et de préférence de 12 à 22 atomes de carbone ; on peut citer par exemple les acides stéarique, palmitique, laurique, caprylique, myristique, alpha ou gamma linolénique, linoléique, arachidonique ou docosopentaénoïque.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcanesulfoniques tels que les acides méthane ou éthanesulfoniques, arylsulfoniques, tels que les acides benzène ou paratoluènesulfoniques et arylcarboxyliques.

Les sels des dérivés de l'invention peuvent être formés avec des bases organiques et minérales. Parmi les bases minérales, ou peut citer les hydroxydes alcalins et alcalinoterreux, tels que par exemple, les hydroxydes de sodium, de potassium, de lithium et de calcium, l'hydroxyde de magnésium ou d'ammonium. Parmi les bases organiques, on peut citer les amines alkylées substituées ou non substituées, telles que par exemple, la triméthylamine, la méthylamine, la propylamine, la N,N-diméthyléthanolamine ou la tris(hydroxyméthyl) méthylamine ; on peut citer également des acides aminés basiques tels que, par exemple, la lysine ou l'arginine ; on peut citer encore d'autres bases telles que, par exemple, la glucosamine ou la procaïne.

Parmi les composés préférés de l'invention, on peut citer les composés de formule (I) dans lesquels U représente un radical :

-CH=CH-CH₂-

ou un radical :

-CH₂-CH=CH-

ainsi que leurs esters avec les alcools renfermant jusqu'à 6 atomes de carbone, leurs amides primaires et secondaires et leurs sels avec les acides organiques ou minéraux ou avec les bases. On peut également citer comme composés préférés, les composés dans lesquels Y est choisi dans le groupe des restes de l'alanine, la proline, la lysine et l'acide alpha ou gamma glutamique et tout spécialement les composés dans lesquels Y représente le reste de l'alanine ainsi que ceux dans lesquels Y représente le reste de la lysine.

L'invention a tout particulièrement pour objet les composés de formule (I), dans lesquels R représente un atome d'hydrogène ou encore ceux dans lesquels R représente un radical méthyle ou dans lesquels R représente un radical -CHF₂, ou encore éthynyle ou vinyle.

Parmi les composés préférés de l'invention, on peut citer les produits dont la préparation est donnée ci-après dans la partie expérimentale et tout particulièrement les produits des exemples 6, 9 et 13. Les amides sont de préférence des amides du type primaire RCONH₂ ou secondaire RCONHalc, alc représentant un radical alkyle renfermant jusqu'à 8 atomes de carbone comme, par exemple, un radical méthyle, éthyle, propyle, isopropyle, butyle.

Les dérivés, objet de la présente invention, possèdent de très intéressantes propriétés pharmacologiques, ils sont doués notamment de remarquables propriétés immunomodulatrices, notamment par activation des monocytes humaines et production de monokines telles le TNF (Tumor Necrosis factor) et l'IL1 (Interleukine 1) seuls ou en présence de l'IFN gamma ainsi que d'intéressantes propriétés anti-bactériennes.

Ces propriétés sont illustrées plus loin dans la partie expérimentale.

Ces propriétés justifient l'utilisation des produits de l'invention ainsi que de leurs sels, à titre de médicaments.

La présente demande a ainsi également pour objet l'application, à titre de médicaments, des dérivés de formule (I) ainsi que de leurs sels d'addition pharmaceutiquement acceptables.

Les médicaments selon l'invention trouvent leur emploi, par exemple, dans le traitement des maladies auto-immunes, qu'il s'agisse d'atteintes non spécifiques d'organes (polyarthrite rhumatoïde, lupus érythémateux, anémie hémolytique, leucopénie auto-immune etc...) ou qu'il s'agisse de maladies spécifiques d'organes (thyroïdite, maladie de Basedow, d'Addison, sclérose en plaques, pemphigus, rectocolite hémorragique, certaines néphropathies, etc...). Ces médicaments peuvent également être utilisés dans le traitement des hémopathies, du cancer, du sida, des affections virales et microbiennes, surtout chroniques et recurrentes (bronchite, grippe etc...) ; des maladies des cavités buccales, etc... Ils peuvent constituer des adjuvants de la thérapie antivirale, de l'antibiothérapie vis-à-vis des germes bactériens et les levures, les champignons (candida albicans...) ou de la chimiothérapie anticancereuse.

Ils trouvent également leur application dans le traitement de nombreux déficits immunitaires secondaires ou acquis observés au cours d'affections très diverses : déficits associés aux troubles métaboliques, déficits d'origine iatrogène, (corticoïdes, radiations ionisantes...) déficits observés chez les grands brûlés, etc.

La dose usuelle, variable selon le dérivé utilisé, le sujet et l'affection en cause peut être par exemple de 0,05 mg à 50 mg par jour. Par voie orale, chez l'homme, le dérivé de l'exemple 8 peut être administré à la dose quotidienne de 0,5 mg à 50 mg, par exemple pour la prévention ou le traitement des infections bactériennes, virales ou fongiques ainsi que pour le traitement des tumeurs d'origine diverses.

Les médicaments selon l'invention trouvent également leur emploi comme antibiotiques dans le traitement des affections bactériennes.

Parmi les médicaments préférés de l'invention, on peut citer ceux qui renferment comme principe actif, les produits des exemples 6, 9 et 13.

Par ailleurs, on a également constaté que l'on obtenait des résultats particulièrement intéressants en associant les produits de formule (I) et l'interféron gamma pour la production par les monocytes de monokines du type TNF, IL₁ par exemple.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides ou les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables, elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), caractérisé en ce que l'on soumet un composé de formule (II) : 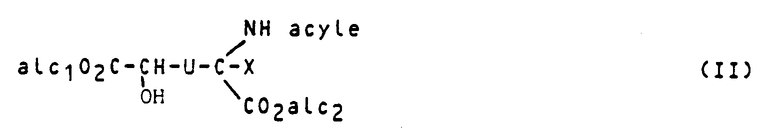
dans laquelle U conserve sa signification précédente, alc₁ et alc₂, identiques ou différents, représentent un radical alkyle renfermant jusqu'à 8 atomes de carbone, X représente soit le radical R, soit un groupement -CO₂alc₃ dans lequel alc₃ représente un radical alkyle renfermant jusqu'à 8 atomes de carbone et acyle représente le reste d'un acide organique renfermant jusqu'à 8 atomes de carbone à l'action d'un halogénure d'alcanesulfonyle ou d'aryl sulfonyle de formule (III) :

HalSO₂alc₄ (III)

dans lequel alc₄ représente un radical alkyle renfermant jusqu'à 8 atomes de carbone ou un radical aryle renfermant jusqu'à 14 atomes de carbone, puis à l'action d'un agent de réduction pour obtenir le composé de formule (IV) : 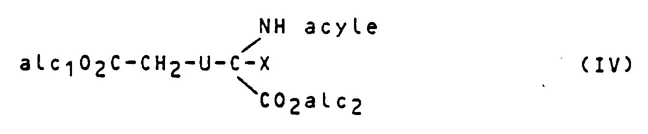
que l'on soumet, si désiré, à la totalité ou à une partie seulement des opérations suivantes dans un ordre quelconque :
a) déprotection de la fonction amine,
b) hydrolyse des fonctions esters, puis le cas échéant décarboxylation lorsque X représente un radical -CO₂alc₃,
c) réduction de la double liaison,
d) estérification ou salification par une base des fonctions carboxy,
e) salification des fonctions amino par un acide,
f) amidification des fonctions aminées libres par un acide aminé ou un peptide constitué de 2, 3 ou 4 acides aminés dont la fonction aminée est protégée puis déprotection de cette fonction aminée.

Dans un mode de réalisation préférée du procédé de l'invention :
- l'halogénure d'alcanesulfonyle ou d'arènesulfonyle est le chlorure de méthanesulfonyle utilisé en présence d'un agent de condensation comme la pyridine ou dans un solvant neutre comme le chlorure de méthylène en présence d'une base comme la triéthylamine,
- l'agent de réduction peut être un métal comme le zinc en présence d'iodure de sodium, dans un solvant basique,
- la déprotection de la fonction amine est réalisée de préférence par action d'un acide minéral dilué tel que l'acide chlorhydrique ou d'un acide organique tel que l'acide trifluoroacétique dans l'alcool benzylique en présence d'un agent tel que le chlorure d'acétyle,
- l'hydrolyse des fonctions esters est réalisée de préférence par saponification à l'aide d'une base minérale comme la soude ou la potasse suivie le cas échéant, d'un traitement par une résine acide.
Lorsque X représente un groupement -CO₂alc₃, l'hydrolyse de la fonction ester est suivie d'une décarboxylation réalisée de préférence à l'acide d'un acide organique tel que l'acide acétique ; elle peut également être effectuée à l'aide d'un acide minéral tel que l'acide chlorhydrique 12N et chauffage dans un solvant el que l'éthanol. Elle peut également intervenir lors du traitement par la résine acide. Une décarbalcoxylation directe peut aussi être réalisée par la procédure de Krapcho ou celle de Keinan et Eren (J. Org. Chem 51 3165-3169 (1986)).
- l'amidification est réalisée en présence d'agents de condensation comme le dicyclohexylcarbodiimide, le N,N′-carbonyl diimidazole ou des amides bis-alkyliques d'acides sulfurés tels que le N,N′-sulfinyl-bis(diméthylamine), SO(N(CH₃)₂)₂, ou encore par formation d'anhydride mixte avec le chloroformiate d'isobutyle.

L'invention a plus précisément pour objet un procédé caractérisé en ce que l'on effectue sur le dérivé de formule : 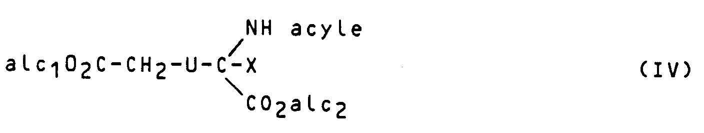
dans laquelle alc₁, alc₂, acyle et X sont définis comme précédemment, les opérations suivantes et dans cet ordre :
a) déprotection de la fonction aminée,
b) hydrolyse des fonctions esters,
pour obtenir le composé de formule (I_{A}) : 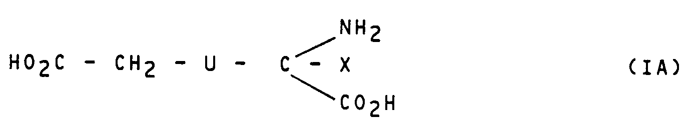
dans laquelle X′ représente soit le radical R soit un groupement -CO₂H, produit (I_{A}) que, si désiré, l'on amidifie par un acide aminé ou un peptide constitué de 2, 3 ou 4 acides aminés dont la fonction aminée est protégée, soumet, le cas échéant à une décarboxylation la fonction X′, puis déprotège la fonction aminée du produit obtenu que l'on réduit, si désiré.

Les différentes étapes de ce procédé sont réalisées selon les modes de réalisation préférés décrits ci-dessus.

L'invention a également pour objet une variante du procédé précédent, caractérisée en ce que l'on soumet le composé de formule : 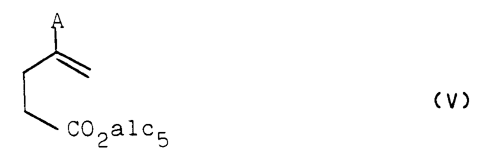
dans laquelle alc₅ représente un radical alkyle renfermant jusqu'à 8 atomes de carbone et A représente un atome d'hydrogène ou un radical méthyle, à l'action d'un composé de formule (VI) : 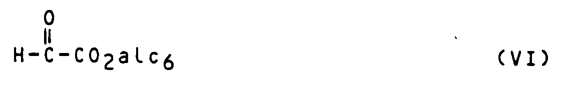
dans laquelle alc₆ représente un radical alkyle renfermant jusqu'à 8 atomes de carbone, pour obtenir le composé de formule (VII) : 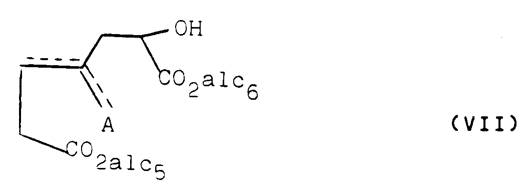
que l'on soumet à l'action d'un halogénure d'alcanesulfonyle ou d'aryl sulfonyle de formule (VIII) :

HalSO₂alc₇ (VIII)

dans lequel alc₇ représente un radical alkyle renfermant jusqu'à 3 atomes ou un radical aryle renfermant jusqu'à 14 atomes de carbone, pour obtenir le composé de formule (IX) : 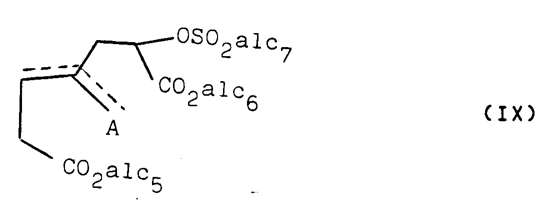
que l'on soumet, à l'action d'un azoture alcalin, pour obtenir le composé de formule (X) : 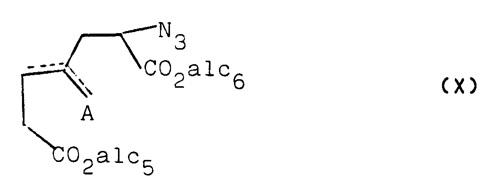
que l'on réduit et soumet le produit ainsi obtenu à une hydrolyse aqueuse, pour obtenir le composé de formule (XI) : 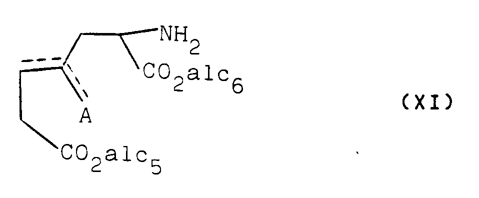
que l'on soumet, si désiré, à la totalité ou à seulement une partie des opérations suivantes dans un ordre quelconque :
a) hydrolyse des fonctions ester,
b) amidification des fonctions amino libres par un acide aminé ou un peptide constitué de 2, 3 ou 4 acides aminés dont la fonction amino est protégée puis déprotection de cette fonction amino,
c) réduction de la double liaison,
d) estérification ou salification par une base des fonctions carboxy,
e) salification par un acide des fonctions amino.

Dans un mode de réalisation préférée :
- la condensation des composés (V) et (VI) a lieu en présence de chlorure ferrique ou de tétrachlorure de titane ou de tout autre acide de Lewis connu ;
- l'halogénure d'alcanesulfonyle ou d'arènesulfonyle est le chlorure de méthanesulfonyle utilisé en présence d'un agent de condensation telle que la pyridine ou d'un solvant neutre comme le chlorure de méthylène en présence d'une base comme la triéthylamine ;
- l'azoture alcalin est l'azoture de sodium ; on peut également utiliser le diphényl phosphorylazide (pour le passage direct de l'alcool à l'azoture) en présence de triphényl phosphine et d'azodicarboxylate d'éthyle ou le phtalimide de potassium dans le diméthylformamide ;
- l'agent de réduction que l'on fait réagir avec le produit de formule (X) est la triphényl phosphine ; on peut également procéder par hydrogénation catalytique, par exemple, en présence de palladium sur charbon actif empoisonné par la quinoléine. L'agent d'hydrolyse du phtalimide pouvant être une base minérale ou l'hydrazine ;
- la suite des opérations auxquelles on soumet le composé de formule (XI) est réalisée de préférence selon les modes de réalisation préférés indiqués ci-dessus pour les différentes étapes de :
   - hydrolyse des fonctions esters,
   - amidification des fonctions amino libres,
   - réduction,
   - estérification et salification.
Les exemples suivants illustrent l'invention sans toutefois la limiter.
Le produit de départ de l'exemple 1 a été préparé comme suit :
**1-(formylamino) 5-hydroxy 3-méthylène 1,1,5-pentane tricarboxylate de triéthyle.**

### Stade 1 : 2-(formylamino) (2-méthyl 2-propényl) propanedioate de diéthyle.

On ajoute 40 g de carbonate de potassium, 0,380 g de catalyseur éther couronne 18 crown 6 et 39,9 g de chlorométhyl propène à une solution de 30 g de formamido-malonate d'éthyle dans 300 cm3 d'acétonitrile, agite au reflux pendant 3 heures, filtre, amène à sec, refroidit à 0°/-5°C, reprend dans 10 cm3 d'éther isopropylique, filtre, lave à l'éther isopropylique, sèche sous pression réduite et obtient 25,2 g du produit attendu. F ≃ 72°C.

### Stade 2 : 1-(formylamino) 5-hydroxy 3-méthylène 1,1,5-pentane tricarboxylate de triéthyle.

On ajoute goutte à goutte en dix minutes une solution de 26,5 g de glyoxylate d'éthyle dans 180 cm3 de chlorure de méthylène, à une solution de 84 g de chlorure ferrique dans 180 cm3 de chlorure de méthylène, agite une heure, refroidit à -20°C, ajoute goutte à goutte en vingt minutes une solution de 34 g de produit tel qu'obtenu au stade 1 dans 180 cm3 de chlorure de méthylène et agite une heure à -20°C. On verse sur 300 cm3 d'eau glacée, extrait au chlorure de méthylène, lave avec de l'acide chlorhydrique 2N puis à l'eau salée, sèche, amène à sec sous pression réduite et obtient 56,5 g du produit attendu F _ 68°C après purification par chromatographie sur silice (éluant : acétate d'éthyle - cyclohexane 6-4).

### Exemple 1 : Acide 2-amino 4-méthylène heptanedioïque.

### Stade A : 1-(formylamino) 3-méthylène 1,1,5-pentane-tricarboxylate de triéthyle.

On dissout 46 g du produit obtenu lors de la préparation décrite ci-dessus dans 500 cm3 de pyridine, refroidit à 0°C et ajoute goutte à goutte, 12 cm3 de chlorure de méthane sulfonyle, agite 3 heures à température ambiante, verse sur 400 cm3 d'acide chlorhydrique 4N glacé et 200 cm3 de chlorure de méthylène, extrait au chlorure de méthylène, lave avec de l'acide chlorhydrique 4N avec une solution saturée de bicarbonate de soude puis avec de l'eau salée, sèche et évapore à sec sous pression réduite. On obtient un produit A.

On laisse au reflux pendant 6 heures une suspension renfermant 10,8 g de produit A, 100 cm3 de diméthoxyéthane, 10 cm3 d'eau, 18,5 g d'iodure de sodium et 16 g de zinc en poudre.

On refroidit la suspension, filtre le précipité, ajoute de l'eau, extrait au chlorure de méthylène, sèche, filtre et amène à sec. On obtient 8,4 g d'une huile que l'on purifie par chromatographie sur silice en éluant par le mélange cyclohexane-acétate d'éthyle (5-5). On obtient 5,75 g de produit recherché.

| Spectre RMN CDCl₃ ppm | |
|---|---|
| H des groupements CO₂CH₂CH₃ | 1,25 (t) 1,27 (t) 4,12 et 4,26 |
| H du groupe formyle | 8,18 |
| H du groupe amino | 7,0 |
| H du CH₂ en position 2 du pentane | 3,14 (s) |
| H du méthylène substitué en position 3 du pentane | 4,8 et 4,9 |
| H des CH₂ en positions 4 et 5 du pentane CH₂-CH₂ | 2,2 à 2,45 |

### Stade B : 1-amino 3-méthylène 1,1,5-pentane-tricarboxylate de triéthyle.

On maintient au reflux, sous agitation pendant 35 minutes, un mélange de 6 g de produit préparé au stade précédent, 60 cm3 d'éthanol et 6 cm3 d'acide chlorhydrique concentré. On évapore la majeure partie de l'éthanol. On refroidit, ajoute de l'eau, neutralise à l'aide de carbonate acide de sodium. On extrait au chlorure de méthylène, lave à l'eau salée, sèche, filtre et amène à sec sous pression réduite.

On obtient ainsi 5,2 g d'une huile que l'on chromatographie sur silice en éluant par le mélange cyclohexane - acétate d'éthyle (7 - 3).

| Spectre RMN CDCl₃ en ppm | |
|---|---|
| H des groupements CO₂CH₂CH₃ | 1,25 (t) et 1,28 (t) 4,12 (q) et 4,23 (q) |
| H du groupe : NH₂ | 2,04 (s) |
| H du CH₂ en position 2 du pentane | 2,80 (s) |
| H du méthylène substitué en position 3 du pentane | 4,99 et 4,95 |
| H des CH₂ en positions 4 et 5 du pentane | 2,28 m et 2,45 m |

### Stade C : acide 2-amino 4-méthylène heptanedioïque.

On ajoute goutte à goutte 9,9 cm3 d'une solution de soude N dans une solution renfermant 945 mg de 1-amino 3-méthylène 1,1,5-pentanetricarboxylate de triéthyle préparé au stade précédent et 10 cm3 d'éthanol. On maintient le mélange réactionnel sous agitation pendant 16 heures à température ambiante et amène à sec sous pression réduite. On reprend le résidu dans 10 cm3 d'eau et ajoute 2 cm3 d'acide acétique pur. On maintient le mélange réactionnel sous agitation à 80°C pendant deux heures et amène à sec. On obtient 830 mg d'un produit que l'on purifie par passage sur résine Dowex 50 W x 8 (éluant eau puis ammoniaque 0,4N). Après passage sur cellulose de 290 mg de résidu (éluant : méthanol -eau (9-1)), on obtient 160 mg du produit recherché.

| Spectre RMN D₂O ppm | |
|---|---|
| H du CH en position 2 de l'acide heptanedioïque | 3,89 (dd J = 4 et 10) |
| H du CH₂ en position 3 de l'acide heptanedioïque | 2,51 (dd) 2,77 (dd) |
| H du méthylène substitué en position 4 de l'acide heptanedioïque | 5,00 et 5,02 |
| H des CH₂ en positions 5 et 6 de l'acide heptanedioïque | 2,3 à 2,5 ppm. |

### Exemple 2 : Acide 2-(L-alanylamino) 4-méthylène heptanedioïque.

### Stade A : 1-[[N-(1,1-diméthyléthoxycarbonyl) L-alanyl] amino] 3-méthylène 1,1,5-pentanetricarboxylate de triéthyle.

On refroidit à 0°C une solution renfermant 945 mg du produit préparé au stade B de l'exemple 1,30 cm3 de diméthoxyéthane, 621 mg de Boc-L-alanine et ajoute par petites fractions en 10 minutes, 678 mg de dicyclohexylcarbodiimide. On laisse sous agitation à 0°C pendant 16 heures. On filtre, et amène le filtrat à sec sous pression réduite. On chromatographie sur silice en éluant par le mélange cyclohexane - acétate d'éthyle (7-3). On obtient 1,3 g du produit recherché.
alpha_{D} = -22°± 2° c = 0,7 % CH₂Cl₂

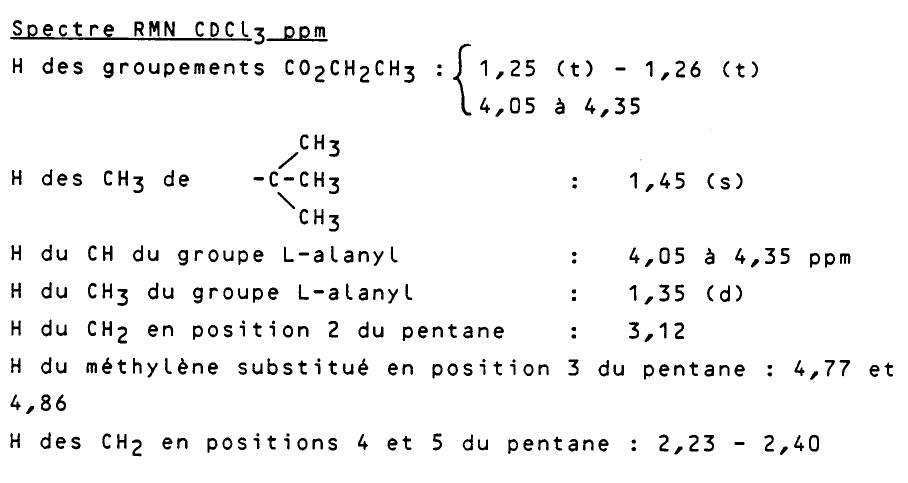

### Stade B : Acide 2-(L-alanyl amino) 4-méthylène heptanedioïque.

On ajoute à 0°C goutte à goutte, 3,9 cm3 d'une solution de soude N dans une solution renfermant 580 mg du produit préparé au stade A et 5 cm3 d'éthanol. On maintient le mélange réactionnel sous agitation pendant 4 heures. On amène à sec sous pression réduite. On reprend le résidu dans l'éthanol. On essore le sel formé, lave et sèche. On obtient 580 mg d'un produit que l'on dissout dans 2 cm3 d'eau.
On ajoute à la solution obtenue de la résine Amberlyst 15 jusqu'à pH = 3. On filtre, lave à l'eau et amène à sec sous pression réduite. On reprend le résidu par 2,5 cm3 d'eau et 4,5 cm3 d'acide acétique. On maintient le mélange réactionnel sous agitation pendant 2 heures à 80°C et amène à sec sous pression réduite. On obtient 370 mg d'une résine que l'on purifie par chromatographie sur silice, en éluant par le mélange propanol-eau (8-2). On obtient 250 mg de l'amine bloquée sous forme de t-butyl carbamate auxquels on ajoute 2,5 cm3 de chlorure de méthylène et 1 cm3 d'acide trifluoroacétique. On laisse 3 heures sous agitation à la température ambiante et amène à sec sous pression réduite. On purifie le résidu obtenu par chromatographie sur silice en éluant par le mélange propanol-eau (8-2). Après passage sur résine Dowex 50 W x 8, on élue par 250 cm3 d'eau distillée puis avec de l'ammoniaque 0,4N et obtient 126 mg du produit recherché.
alpha _{D} = + 3° ± 1,5 (c = 0,6 % H₂O)

| Spectre RMN D₂O ppm | |
|---|---|
| H du CH₃ du groupe L-alanyl | 1,49 (d) dédoublé |
| H du CH du groupe L-alanyl | 4,09 (q) dédoublé |
| H du CH en position 2 de l'acide heptanedioïque | 4,36 |
| H du méthylène substitué en position 4 de l'acide heptanedioïque | 4,85 et 4,89 |
| H des CH₂ en positions 5 et 6 de l'acide heptanedioïque | 2,2 à 2,7. |

### Exemple 3 : Acide 2-[(gamma-D glutamyl)amino] 4-méthylène heptanedioïque.

### Stade A : 3-méthylène 1-[[O-méthyl N-(trifluoroacétyl) gamma-D glutamyl]amino] 1,1,5-pentanetricarboxylate de triéthyle.

On refroidit à 0°C, une solution renfermant 1,27 g du produit préparé au stade B de l'exemple 1, 50 cm3 de diméthoxyéthane et 1,22 g d'acide (N-trifluoroacétyl)-D-glutamate de 1-méthyle. On ajoute par petites fractions 1,06 g de dicyclohexylcarbodiimide et laisse 16 heures sous agitation à 0°C. On filtre le précipité formé et amène à sec sous pression réduite. On obtient 2,4 g de résidu que l'on chromatographie sur silice en éluant par le mélange cyclohexane - acétate d'éthyle (6-4). On obtient 1,6 g du produit recherché (rf. = 0,3).
alpha_{D} = + 11°5 ± 1,5°
(c = 0,65% CH₂Cl₂)

| Spectre RMN CDCl₃ ppm | |
|---|---|
| H des groupements CO₂CH₂CH₃ | 1,18 à 1,31 4,11 (q) et 4,25 (q) |
| H du CH₃ de CO₂CH₃ | 3,77 (s) |
| H de CH du gamma-glutamyl | 4,50 (dt) |
| H du CH₂ en position 2 du pentane | 3,11 (s) |
| H du méthylène substitué en position 3 du pentane | 4,75 et 4,87 |
| H des CH₂ en positions 4 et 5 du pentane | 2,05 à 2,50. |

### Stade B : Acide 2-[(gamma-D-glutamyl)amino] 4-méthylène heptanedioïque.

On ajoute à 0°C 11,8 cm3 de soude N dans une solution renfermant 1,1 g du produit préparé au stade A et 50 cm3 d'éthanol. On laisse le mélange réactionnel sous agitation pendant 16 heures. On amène à sec et obtient un résidu que l'on reprend avec 10 cm3 d'eau et 2 cm3 d'acide acétique pur. On maintient le mélange sous agitation pendant 4 heures à 80°C. On mène à sec sous pression réduite. On obtient 1,8 g d'un produit que l'on purifie sur silice en éluant par le mélange éthanol - ammoniaque (90 - 10). On isole 510 mg du produit recherché brut que l'on purifie en le reprenant dans l'éthanol jusqu'à l'obtention du précipité blanc. On essore et sèche sous pression réduite. On obtient un produit que l'on dissout dans l'eau, filtre et lyophilise. On obtient après 2 lyophilisations successives 280 mg du produit recherché.
alpha_{D} = -12° ± 1,5° (c = 0,7% H₂O)

| Spectre RMN D₂O ppm | |
|---|---|
| H du méthylène substitué en position 4 de l'acide heptanedioïque | 4,86 - 4,88 |
| H du CH en position 2 de l'acide heptanedioïque | 4,33 |
| H du CH du gamma-D-glutamyl | 3,76 |
| H des CH₂ en positions 5 et 6 de l'acide heptanedioïque | 2,05 à 2,70 |

### Exemple 4 : Acide 2-amino 3-heptènedioïque.

### Stade A : 1-(formyl amino) 2-penten-1,1,5-tricarboxylate de triéthyle.

On ajoute 4,2 g d'iodure de sodium et 3,7 g de zinc dans une solution de 2,4 g du produit A obtenu au stade A de l'exemple 1. On porte le milieu réactionnel dans le diméthoxy éthane au reflux en présence d'eau sous agitation pendant 2 heures. On ajoute 50 ml d'eau. On filtre et extrait au chlorure de méthylène. On sèche et amène à sec sous pression réduite. On obtient 2,3 g de produit brut que l'on chromatographie sur silice en éluant par le mélange cyclohexanne - acétate d'éthyle (5-5). On obtient ainsi 1,6 g du produit recherché.

| Spectre RMN | |
|---|---|
| H du formyle | 8,1 ppm |
| H éthylénique en position 2 | 6,0 - 6,3 ppm |
| H éthylénique en position 3 | 5,7 ppm |
| H des CH₂ en positions 4 et 5 | 2,3 à 2,5 ppm |

### Stade B : Acide 2-amino 3-heptènedioïque.

On ajoute 0,25 cm3 d'acide chlorhydrique 12N dans une solution renfermant 0,5 g du produit préparé au stade A dans 5 cm3 d'éthanol. On porte le milieu réactionnel au reflux pendant une heure. On laisse refroidir à la température ambiante et ajoute 20 cm3 d'eau. On chasse l'éthanol sous pression réduite. On amène à neutralité à l'aide d'une solution de bicarbonate de potassium à 10%. On extrait à l'acétate d'éthyle. On sèche les phases organiques sur sulfate de magnésium et évapore à sec. On obtient 0,4 g de produit brut.

On dissout 0,34 g du produit ainsi obtenu dans 10 cm3 d'éthanol à 0°C et ajoute goutte à goutte 3,2 cm3 d'une solution de soude N. On laisse le milieu réactionnel sous agitation pendant 16 heures à la température ambiante. On amène à sec. On reprend le résidu par 5 cm3 d'eau. On ajoute 0,2 cm3 d'acide acétique. On abandonne la solution obtenue pendant 16 heures sous agitation à la température ambiante et amène à sec.

On purifie le produit obtenu par passage sur cellulose (éluants : propanol - eau 9-1, 8-2 et 7-3), filtration sur gel d'exclusion, passage sur résine échangeuse d'ions. On obtient 0,110 g du produit recherché.

| Spectre RMN D₂O | |
|---|---|
| H du CH en position 2 | 4,20 |
| H éthyléniques | 5,59 dd j = 8 et 15,5 |
| | 5,98 d j = 15,5 et 6. |
| H des CH₂ en positions 5 et 6 | 2,25 à 2,42. |

### Exemple 5 : Acide 6-amino 3-heptènedioïque.

### Stade A : 6-hydroxy 3-heptènedioate de (7) éthyle et de (1) méthyle.

On ajoute en 15 minutes, à 0°/+6°C, une solution de 40 g de 4-penténoate de méthyle dans du chlorure de méthylène, dans une suspension renfermant 71,5 g de glyoxylate d'éthyle dans un litre de chlorure de méthylène et 115 g de chlorure ferrique. On laisse en contact pendant 15 minutes à 0° + 5°C. On laisse remonter à la température ambiante. On verse sur de la glace. On décante et lave la phase CH₂Cl₂ avec de l'acide chlorhydrique N. On sèche sur sulfate de magnésium. On évapore et obtient 84 g de produit brut.

On purifie sur silice (éluant : cyclohexane - acétate d'éthyle (7-3).

On obtient le produit recherché. Rendement 70 %.

### Stade B : 6-[méthyl sulfonyl)oxy] 3-heptènedioate de (7) éthyle et de (1) méthyle.

On dissout 1,5 g du produit préparé au stade A dans 15 cm3 de chlorure de méthylène à la température ambiante. On ajoute 15,6 cm3 de pyridine et 0,7 cm3 de chlorure de méthane sulfonyle. On agite 16 heures à la température ambiante. On verse lentement le milieu réactionnel sur une solution d'acide chlorhydrique 6N additionnée de chlorure de méthylène. On extrait au chlorure de méthylène. On lave à l'aide d'une solution de carbonate acide de sodium. On sèche et évapore à sec. On purifie le résidu par chromatographie sur silice en éluant par le mélange chlorure de méthylène - acétate d'éthyle (9-1).

On réunit et amène à sec les fractions contenant le produit désiré. On obtient 0,3 g du produit recherché.
Rf. = 0,45 dans le système CH₂Cl₂ - AcOEt (85-15).

| Spectre RMN (ppm) | |
|---|---|
| H de SO₂CH₃ | 3,15 |
| H de CO₂CH₂CH₃ | 1,31 et 4,26 |
| H du CH en position 6 | 5,05 |
| H de CH₂ en position 5 | 2,55 à 2,8 |
| H éthyléniques | 5,56 et 5,74 |
| H de CO₂CH₃ | 3,68 |
| H du CH₂ en position 2 | 3,08 |

### Stade C : 6-azido 3-heptènedioate de (7) éthyle et de (1) méthyle.

On dissout 1,6 g du produit préparé au stade précédent dans 20 cm3 de diméthyl formamide, on ajoute 0,39 g d'azoture de sodium NaN₃ et agite 16 heures à la température ambiante. On évapore le diméthyl formamide à 35°C maximum et reprend le résidu obtenu par le chlorure de méthylène. On lave à l'acide d'une solution de carbonate acide de sodium à 10 % puis avec de l'eau salée. On sèche et évapore à sec. On obtient 1,25 g de résidu que l'on chromatographie sur silice en éluant par le mélange acétate d'éthyle - cyclohexane (3-7). On amène à sec les fractions intéressantes. On obtient 1,03 g du produit recherché. Rf. = 0,3 dans un mélange cyclohexane - acétate d'éthyle (8-2).

| Spectre RMN. | |
|---|---|
| H éthyléniques | 5,55 et 5,75 ppm |
| H de CO₂CH₃ | 3,69 ppm |
| H du CH₂ en position 2 | 3,08 ppm |
| H du CH₂ en position 5 | 2,45 à 2,65 ppm |
| H de CO₂Et | 1,31 ppm et 4,25 ppm |
| H du CH en position 6 | 3,88 ppm |

### Stade D : 6-amino 3-heptènedioate de (7) éthyle et de (1) méthyle.

On dissout 21,1 g du produit obtenu au stade précédent dans 350 cm3 de tétrahydrofuranne. On refroidit à 0°C et ajoute 27,5 g de triphényl phosphine. On agite à la température ambiante pendant 5 heures. On ajoute 23,6 cm3 d'eau et agite à la température ambiante pendant 16 heures. On évapore le tétrahydrofuranne. On reprend le résidu dans le chlorure de méthylène et extrait deux fois à l'aide d'une solution d'acide chlorhydrique 2N. On neutralise avec du carbonate acide de sodium et extrait au chlorure de méthylène. On lave à l'eau salée, sèche et évapore à sec. On obtient 16,2 g du produit recherché.
Rf. = 0,25 dans acétate d'éthyle - éthanol (9-1).

| Spectre RMN CDCl₃ | |
|---|---|
| H de NH₂ | 1,61 ppm |
| H du CH en position 6 | 3,51 ppm |
| H de CO₂Et | 1,28 et 4,18 ppm |
| H du CH₂ en position 5 | 5,52 et 5,68 ppm |
| H du CH₂ en position 2 | 3,07 ppm. |

### Stade E : Acide 6-amino 3-heptènedioïque.

On dissout 1 g du produit obtenu au stade précédent dans 10 cm3 d'éthanol. On ajoute 5,1 cm3 d'une solution de soude 2N. On agite 16 heures à la température ambiante. On évapore à sec sous pression réduite. On désalcalinise le produit obtenu sur résine Dowex 50 W x 8. On évapore à sec les fractions contenant le produit désiré. On obtient un produit que l'on lyophilise dans 150 cm3 d'eau bidistillée filtrée. On obtient 660 mg du produit recherché. Rf. = 0,5 dans le mélange BuOH-AcOH-H₂O (4-2-2).

| Spectre RMN D₂O | |
|---|---|
| H éthyléniques | 5,46 ppm - 5,80 ppm |
| H du CH₂ en position 5 | 2,49 à 2,76 ppm |
| H du CH en position 6 | 3,79 ppm |
| H du CH₂ en position 2 | 2,98 ppm |

### Exemple 6 : Acide 6-(L-alanylamino) 3-heptènedioïque.

### Stade A : 6-[[N-(1,1-diméthyléthoxy)carbonyl] L-alanylamino] 3-heptènedioate de (7) éthyle et de (1) méthyle.

On dissout 3,9 g du produit obtenu au stade D de l'exemple 5 dans 200 cm3 de diméthoxy éthane. On ajoute 3,63 g de N-Boc L-alanine. On refroidit à -5° 0°C. On ajoute 3,99 g de dicyclohexylcarbodiimide et agite vigoureusement 16 heures à 0°C. On filtre et évapore à sec. On reprend dans du diméthoxy éthane et filtre. On évapore à sec et reprend dans le chlorure de méthylène. On lave avec une solution saturée de bicarbonate de soude puis avec de l'eau salée, sèche et évapore à sec. On obtient 7,7 g du produit recherché brut que l'on chromatographie sur silice en éluant par le mélange cyclohexane - acétate d'éthyle (7-3). On obtient 5,4 g du produit recherché.
alpha_{D} = -18,5 ± 1° (c = 1,6% CH₂Cl₂)

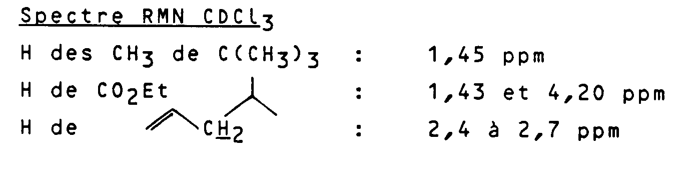

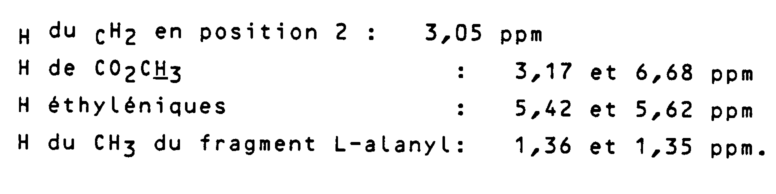

### Stade B : Acide 6-(L-alanylamino) 3-heptènedioïque.

On dissout 790 mg du produit obtenu au stade A dans 20 cm3 d'éthanol à la température ambiante.

On refroidit au bain de glace et ajoute 2,4 cm3 d'une solution de soude 2N. On agite à la température ambiante pendant 2 heures 30. On amène à pH 6 à l'aide d'une solution concentrée d'acide chlorhydrique et évapore à sec. On reprend le résidu obtenu dans 10 cm3 de dioxanne et ajoute 1 cm3 d'une solution d'acide chlorhydrique 12N.

On agite la suspension obtenue pendant 30 minutes à 70°C. On filtre le précipité et évapore à sec le filtrat. On reprend le résidu par 2 cm3 d'eau, amène le pH à 6 avec de la soude N, passe sur résine échangeuse d'ions (H⁺) élue à l'eau puis à l'ammoniaque 0,5N et évapore à sec les fractions contenant le produit désiré. On obtient 420 mg d'un produit que l'on reprend dans l'eau bidistillée, filtrée et lyophilise. On obtient 370 mg du produit recherché.
Rf. = 0,5 en éluant avec le mélange BuOH-AcOH-H₂O (4-2-2).
alpha_{D} = -4° ± 1° (c = 1% HCl5N)

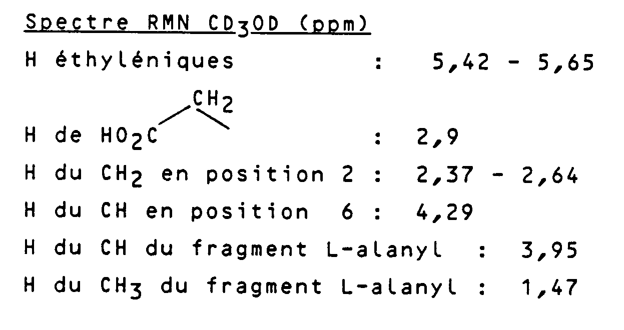

### Exemple 7 : 6-[[N-[(1-oxo octadécyl) L-alanyl] 0-méthyl gamma-D-glutamyl] amino] 3-heptènedioate de (7) éthyle et de (1) méthyle.

On refroidit à 10-12°C une solution renfermant 1,99 g de [N-(1-oxo octadécyl) L-alanyl] D-glutamate de 1 méthyle et 70 cm3 de tétrahrdrofuranne anhydre. On ajoute 1,16 cm3 de triéthylamine et 0,6 cm3 de chloroformiate d'isobutyle. On laisse sous agitation à 10 12°C pendant 35 minutes et ajoute 0,86 g du produit obtenu au stade D de l'exemple 5 et 10 cm3 de tétrahydrofuranne anhydre.

On laisse sous agitation à la température ambiante pendant 2 heures. On ajoute de la glace, extrait au chlorure de méthylène, lave à l'aide d'une solution d'acide chlorhydrique N et à l'eau salée, sèche et évapore à sec sous pression réduite. On obtient 2,9 g du produit recherché brut que l'on purifie par chromatographie sur silice en éluant par le mélange CH₂Cl₂-MeOH (98-2). On obtient 1,8 g de produit recherché.
F = 110°C.
alpha_{D} = -14,5° ± 2° CH₂Cl₂ c = 0,5%

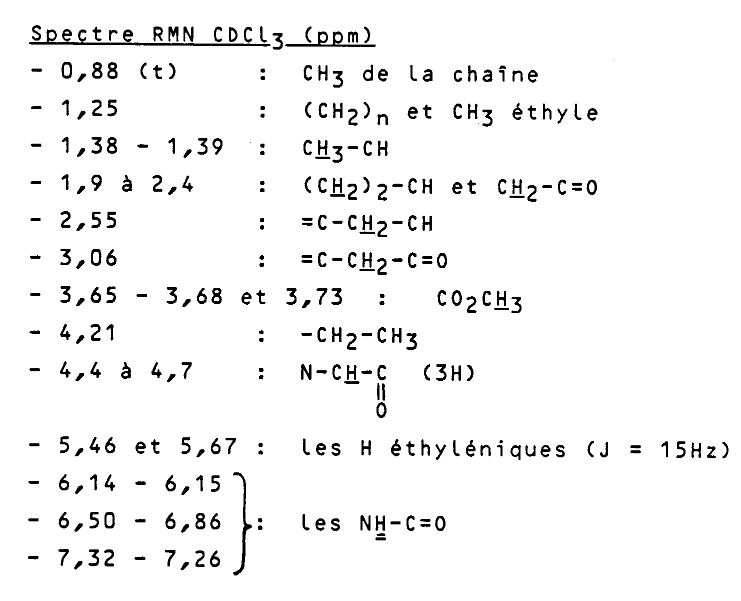

### Exemple 8 : Acide 6-[[N-[(1-oxo octadécyl) L-alanyl] gamma-D-glutamyl] amino] 3-heptènedioïque (sel de sodium).

On ajoute à 0°C, 3,6 cm3 d'une solution de soude N dans une solution renfermant 800 mg du produit obtenu à l'exemple 7 et 15 cm3 d'éthanol. On maintient le mélange réactionnel sous agitation pendant 2 heures à la température ambiante. On amène à sec sous pression réduite sans chauffer. On reprend le résidu obtenu dans l'eau, filtre et lyophilise. On obtient 775 mg du produit recherché fondant à 200°C (P.F. peu net).
alpha_{D} = -26° ± 2° (c = 0,5% H₂O)

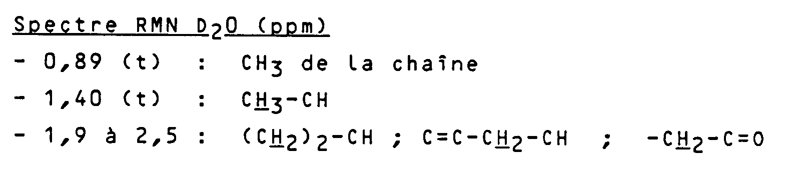

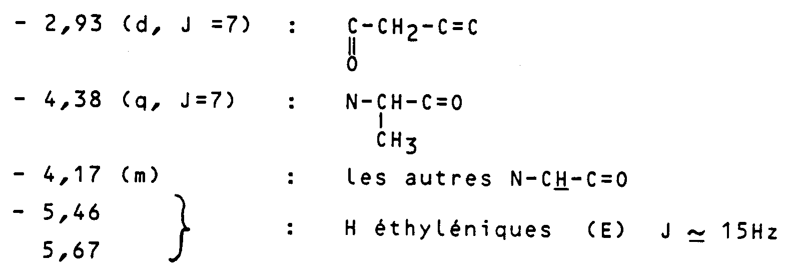

### Exemple 9 : Acide 6-(L-lysyl amino) 3-heptènedioïque.

### Stade A : N,N′-bis[(1,1-diméthyléthoxy) carbonyl] L-lysine.

On dissout 1,46 mg de lysine dans une solution de 0,4 g de soude en pastilles dans 1 cm3 d'eau. On ajoute 2 cm3 de tert-butanol. On introduit goutte à goutte le (BOC)₂O. On agite la suspension obtenue pendant 4 heures à la température ambiante. On dilue à l'eau, amène à pH 4 au moyen d'une solution d'hydrogénosulfate de potassium (10 g/300 cm3 d'eau). On extrait à l'acétate d'éthyle, lave à l'eau salée jusqu'à neutralité, sèche et évapore à sec. On obtient 2,35 g de produit recherché.
alpha_{D} = +12° ± 1° (c = 1% H₂o)

| Spectre RMN CDCl₃ | |
|---|---|
| H des groupes tert-butyles | 1,45 - 1,47 - 1,52 ppm |
| H du CH₂ en position 6 de la lysine | 3,12 ppm |
| H du CH en position 2 de la lysine | 4,12 et 4,30 ppm |

### Stade B : 6[[N,N′-bis[(1,1-diméthyléthoxy) carbonyl] L-lysyl] amino] 3-heptènedioate de (7) éthyle et de (1) méthyle.

On dissout 1,74 g du produit préparé au stade A et 1,076 g de 6-amino 3-heptène dioate de (7) éthyle et de (1) méthyle dans 80 cm3 de diméthoxyéthane. On refroidit à 0°C et introduit une solution de 1,03 g de dicyclohexylcarbodiimide dans 20 cm3 de diméthoxyéthane. On agite pendant 16 heures à 0°C. On filtre et amène à sec le filtrat. On obtient 2,9 g de produit recherché brut que l'on purifie par chromatographie sur silice en éluant par le mélange acétate d'éthyle - cyclohexane (1-1). On amène à sec les fractions contenant le produit et obtient 1,78 g de produit recherché.
alpha_{D} = -14° ± 2° (c = 0,65% CH₂Cl₂)

| Spectre RMN CDCl₃ (ppm) | |
|---|---|
| H de C(CH₃)₃ | 1,44 - 1,45 |
| H de CO₂CH₃ | 3,68 - 3,69 |
| H de CO₂CH₂CH₃ | 4,20 (m) |
| H de CO₂CH₂CH₃ | 1,28 (t) |
| H du CH₂ en position 2 du fragment heptènedioïque | 3,05 (d) |
| H du CH₂ en position 5 du fragment heptènedioïque | 2,55 (m) |
| H des CH en position 2 du fragment lysyl et en position 6 du fragment heptènedioïque | 4,1 - 4,60. |

### Stade C : Sel de sodium de l'acide 6-[[N,N'-bis[(1,1-diméthyl éthoxy) carbonyl] L-lysyl] amino] 3-heptènedioïque.

On ajoute à 0°C, 6 cm3 d'une solution de soude N dans une solution de 1,47 g du produit préparé au stade précédent et 25 cm3 d'éthanol. On agite une heure trente minutes à 0°C puis deux heures trente à température ambiante. On évapore à la température ambiante sous pression réduite. On obtient 1,57 g du produit recherché.

| Spectre RMN D₂O (ppm) | |
|---|---|
| H des C(CH₃)₃ | 1,43 - 1,45 |
| H des CH₂ en position 6 des fragments lysyl | 3,08 (t) |
| H des CH en position 2 des fragments lysyl et en position 6 du fragment heptènedioïque | 4,03 (m) - 4,22 (m) |
| H éthyléniques | 5,14 (m) - 5,60 (m) |
| H du CH₂ en position 2 du fragment heptènedioïque | 2,93 (d) |

### Stade D Acide 6-(L-lysyl amino)-3-heptènedioïque

On dissout 1,16 g du produit obtenu au stade précédent dans 17 cm3 de dioxanne. On ajoute 1,7 cm3 d'une solution d'acide chlorhydrique concentrée. On porte à 70°C pendant 30 minutes. On évapore à sec. On dilue à l'eau, amène à pH 6 par addition de bicarbonate de sodium. On purifie par passage sur résine échangeuse d'ions Dowex 50 W x 8 et élue à l'eau puis à l'ammoniaque 0,2N. On évapore à sec et lyophilise. On obtient 536 mg du produit recherché.
alpha_{D} = + 15° ± 1° c = 1% H₂O

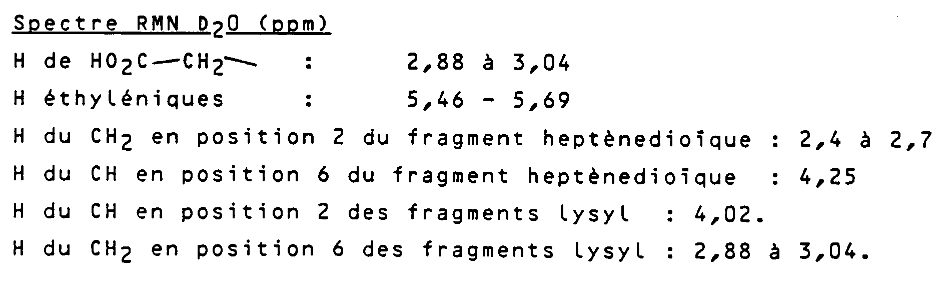

### Exemple 10 : Acide 6-[(L-alanyl gamma-D-glutamyl)amino] 3-heptènedioïque.

### Stade A : 6-[[N-[N-trifluoroacétyl)L-alanyl] (0-méthyl) gamma-D-glutamyl] amino] 3-heptènedioate de (7) éthyle et de (1) méthyle.

On ajoute à 12°C, 0,62 cm3 de chloroformiate d'isobutyle dans une solution renfermant 1,44 g de N-[N-(trifluoroacétyl) L-alanyl] D-glutamate de 1-méthyle, 1,2 cm3 de triéthylamine et 70 cm3 de tétrahydrofuranne. On maintient sous agitation pendant une heure. On ajoute goutte à goutte une solution de 0,9 g dans 30 cm3 de tétrahydrofuranne du produit obtenu au stade D de l'exemple 5. On maintient la solution obtenue sous agitation pendant une heure à la température ambiante. On ajoute 20 cm3 d'eau et extrait au chlorure de méthylène. On lave la phase chlorométhylénique avec 20 cm3 d'acide chlorhydrique N, sèche, amène à sec. On obtient 2,2 g d'un produit que l'on purifie par chromatographie sur silice en éluant dans le mélange chlorure de méthylène - acétate d'éthyle (4-6). On obtient 1,32 g de produit qu'on empâte à l'éther isopropylique et obtient 1,12 g du produit recherché.
F = 70°C.

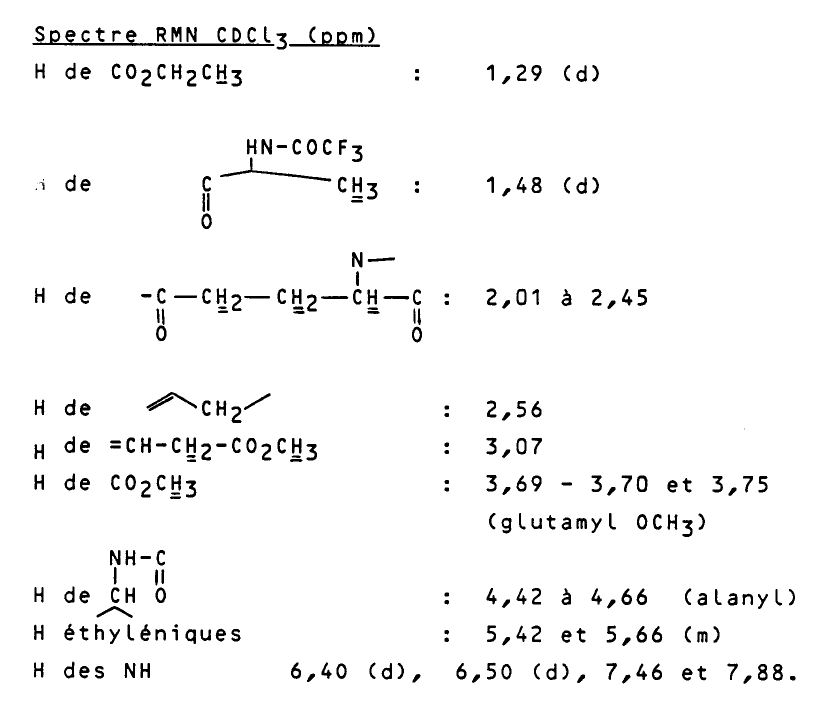

### Stade B : Acide 6[(L-alanyl gamma-D-glutamyl) amino] 3-heptènedioïque.

On ajoute goutte à goutte 6,4 cm3 d'une solution de soude N dans une solution renfermant 0,8 g du produit préparé au stade précédent et 16 cm3 d'éthanol à 0°C. On laisse la solution obtenue sous agitation pendant 16 heures à la température ambiante. On amène à pH 7 avec de l'acide acétique. On concentre la solution sous pression réduite, ajoute 5 cm3 d'eau au résidu et purifie par passage sur résine échangeuse d'ions Dowex 50 W x 8 ; on élue avec de l'ammoniaque 0,2N, lyophilise et obtient 0,545 g du produit recherché.
alpha_{D} = +4° ± 2° (c = 1% H₂O)

| Spectre RMN | |
|---|---|
| H du CH₃ du fragment alanyl | 1,47 (d) - 1,49 (d) |
| H du CH₂ en position 2 du fragment heptènedioïque | 2,91 (d) |
| H du CH₂ en position 5 du fragment heptènedioïque et des CH₂ du fragment glutamyl | 1,89 à 2,60 |
| H du CH du fragment alanyl | 3,94 |
| H du CH du fragment glutamyl | 4,15 à 4,30 |
| H éthyléniques | 5,45 et 5,66 |

### Exemple 11 : Acide 2-amino 2-méthyl 4-méthylène heptanedioïque.

### Stade A : (2-méthyl 2-propényl) propanedioate de diéthyle.

On dissout 50 g de 2-méthyl malonate de diéthyle dans 500 cm3 d'acétonitrile, ajoute 80 g de carbonate de potassium, 800 mg d'éther couronne (18 crown 6) puis 75 g de 3-chloro 2-méthyl 1-propène, agite et porte au reflux pendant 8 heures. On filtre l'insoluble et concentre à sec le filtrat. On chromatographie le résidu sur silice, élue par un mélange cyclohexane - acétate d'éthyle (9-1) et obtient 28,3 g de produit pur et 32 g de mélange. On chromatographie ce dernier sur silice en éluant par un mélange cyclohexanne - acétate d'éthyle (9-1) et isole encore 16,5 g de produit attendu.
Rf. = 0,3 dans cyclohexane - acétate d'éthyle (9-1).

### Stade B : méthyl (2-méthyl 2-propényl) propanedioate de monoéthyle.

On refroidit à 0°C, 27 g de méthyl (2-méthyl 2-propényl) propanedioate de diéthyle dans 250 cm3 d'éthanol, ajoute goutte à goutte 65 cm3 de soude 2N et agite 24 heures à température ambiante.

On verse le mélange réactionnel sur 30 cm3 d'acide chlorhydrique 6N glacé, extrait à l'acétate d'éthyle et évapore à sec. On reprend le résidu par du chlorure de méthylène, extrait avec une solution aqueuse à 10% de bicarbonate de soude, acidifie avec de l'acide chlorhydrique concentré et extrait à l'acétate d'éthyle. On lave la phase organique à l'eau salée, sèche, évapore à sec et obtient 24 g de produit attendu. Rf = 0,4 dans CH₂Cl₂-MeOH (9-1).

### Stade C : 2,4-diméthyl 2-[(méthoxycarbonyl) amino] 4-pentènoate d'éthyle.

On dissout 27,5 g de produit obtenue comme ci-dessus dans 150 cm3 de chlorure de thionyle, agite 3 heures au reflux et amène à sec sous pression réduite et obtient 30 g de chlorure d'acide. On dissout 14,5 g de ce dernier dans 100 cm3 d'acétone, refroidit à 0°C, ajoute goutte à goutte 5,187 g d'azoture de sodium en solution dans 40 cm3 d'eau, agite une heure à 0°C. On évapore l'acétone, extrait à l'éther, sèche, évapore à sec et obtient 25 g d'azoture.

On dissout ce dernier dans 200 cm3 ce méthanol, agite au reflux pendant 16 heures, évapore à sec, reprend le résidu par du chlorure de méthylène, lave la phase organique à l'eau salée, sèche, évapore à sec et obtient 22 g de produit brut. On chromatographie celui-ci sur silice en éluant avec un mélange cyclohexane - acétate d'éthyle (8-2) et isole 11,2 g de produit attendu.
Rf. = 0,4 dans cyclohexane - acétate d'éthyle (8-2).

### Stade D : 6-hydroxy 2-[(méthoxycarbonyl) amino] 2-méthyl 4-méthylène heptanedioate de diéthyle.

A une suspension de 30,5 g de chlorure ferrique dans 150 cm3 de chlorure de méthylène, on ajoute goutte à goutte 9,9 g de glyoxylate d'éthyle 150 cm3 de chlorure de méthylène et agite 30 minutes à température ambiante. On refroidit à -60°C et ajoute goutte à goutte 11,1 g de produit obtenu en C dans 150 cm3 de chlorure de méthylène. On laisse remonter à -30°C. On verse le mélange réactionnel sur 400 cm3 d'eau glacée et extrait au chlorure de méthylène. On lave la phase organique à l'eau avec une solution aqueuse à 10% de bicarbonate de sodium puis à l'eau salée, sèche et évapore à sec. On obtient 15,3 g de produit brut correspondant à un mélange : 65% du produit attendu et 35% du produit 4-méthyle heptène. Après passage sur silice (éluant : cyclohexane - acétate d'éthyle (7-3), on obtient 11,4 g du mélange purifié.
Rf. = 0,25 dans cyclohexane - acétate d'éthyle (6-4).

### Stade E : 2-[(méthoxycarbonyl) amino] 2-méthyl 4-méthylène 6-[(méthylsulfonyl) oxy] heptanedioate d'éthyle.

On dissout 11,4 g de produit obtenu précédemment dans 150 cm3 de pyridine, refroidit à 0°C et ajoute 4,9 g de chlorure de méthanesulfonyle, agite une heure à 0°C et 5 heures à température ambiante. On verse sur de l'acide chlorhydrique 6N, extrait à l'acétate d'éthyle, lave la phase organique à l'acide chlorhydrique 6N, avec une solution aqueuse à 10% de bicarbonate de soude puis à l'eau salée. On sèche et évapore à sec. On chromatographie le résidu sur silice, élue avec un mélange chlorure de méthylène - acétate d'éthyle (9-1) et sépare 6 g de produit attendu, 6 g de mélange de "4-méthylène heptane" et "4-méthyl heptène" et 0,7 g de "4-méthyl heptène". Après une 2ème chromatographie dans les mêmes conditions de la fraction mélange, on isole 3,5 g de produit attendu, 1 g de mélange et 1,1 g de produit 4-méthyl heptène.
Rf. dans CH₂Cl₂ - acétate d'éthyle 9-1 :
0,4 pour le produit 4-méthylène heptane,
0,35 pour le produit 4-méthyl heptène.

### Stade F : 2-[(méthoxycarbonyl) amino] 2-méthyl 4-méthylène heptanedioate de diéthyle.

On dissout 1,5 g de produit obtenu ci-dessus dans 20 cm3 de diméthoxyéthane, on ajoute successivement 2 cm3 d'eau, 2,75 g d'iodure de sodium et 2,4 g de zinc, agite et porte au reflux pendant 8 heures. On filtre, lave l'insoluble à l'eau, au chlorure de méthylène, extrait le filtrat au chlorure de méthylène, lave la phase organique à l'eau salée, sèche et évapore à sec. On chromatographie le résidu sur silice, élue par un mélange cyclohexane - acétate d'éthyle (8-2) et isole 0,85 g de produit attendu.
Rf. = 0,25 dans cyclohexane - acétate d'éthyle (8-2).

### Stade G : Acide 2-amino 2-méthyl 4-méthylène heptanedioïque.

On dissout 420 mg de produit obtenu au stade F dans 5 cm3 d'éthanol, ajoute 1,66 cm3 de soude 2N et agite 2 heures à température ambiante. On évapore l'éthanol, reprend le résidu par 5 cm3 de soude 2N, agite et porte au reflux pendant 20 heures. On concentre à sec, reprend dans l'eau et neutralise avec de l'acide chlorhydrique 2N. On passe la solution sur résine échangeuse d'ions Dowex 50 W x 8(H⁺), élue à l'eau puis à l'ammoniaque 0,7N. On concentre à sec les fractions intéressantes, reprend dans 10 cm3 d'eau, lyophilise 16 heures et obtient 250 mg de produit attendu.
Rf. = 0,5 dans BuOH - AcOH - H₂O (4-2-2).

| Spectre RMN (D₂O) ppm | |
|---|---|
| 3,85 | du CH₂ en position 6 |
| 5,17 - 5,23 et 5,22 - 5,27 | H du méthylène substitué en position 4 |
| 1,54 | H du CH₃ substitué en position 2 |
| 2,4 à 2,9 | les CH₂ |

### Exemple 12 : acide 2-amino 2-(difluorométhyl) 4-méthylène heptanedioïque.

### Stade A : (2-méthyl 2-propényl) propanedioate de (1) (1,1-diméthyléthyle) et de (3) éthyle.

On dissout 33 g de malonate d'éthyle et de terbutyle dans 400 cm3 d'acétonitrile, ajoute en agitant 29 g de carbonate de potassium, 0,5 g d'éther couronne (18-6) et 300 g de 3-chloro 2-méthyl 1-propène puis agite 16 heures à 65°C. On filtre l'insoluble et évapore à sec le filtrat. On chromatographie le résidu sur silice, élue par un mélange cyclohexane - acétate d'éthyle (95-5) et isole 18 g de produit pur et 22g de mélange. On chromatographie celui-ci sur silice élue par un mélange cyclohexane - acétate d'éthyle (97,5 - 2,5) et isole encore 11 g de produit.
Rf. = 0,35 dans cyclohexane - acétate d'éthyle (95-5).

### Stade B : (Difluorométhyl) (2-méthyl 2-propényl) propane dioate de (1) (1,1-diméthyléthyl) et de (3) éthyle.

On met en suspension 2,8 g d'hydrure de sodium dans 100 cm3 de tétrahydrofuranne, ajoute goutte à goutte 11,1 g de produit obtenu au stade A dans 100 cm3 de tétrahydrofuranne et agite une heure à 42°C. On fait barboter du Fréon 22 pendant 15 minutes en agitant à 45°C et agite encore une heure à 45°C et une heure à température ambiante sous atmosphère de Fréon 22. On hydrolyse le milieu réactionnel avec de l'eau salée, extrait au chlorure de méthylène, lave la phase organique à l'eau salée, sèche et concentre à sec. On obtient 13 g de produit attendu.
Rf. = 0,4 dans cyclohexane - acétate d'éthyle (95-5).

### Stade C : (Difluorométhyl) (2-méthyl 2-propényl) propane dioate de monoéthyle.

On dissout 13 g de produit obtenu ci-dessus dans 100 cm3 de chlorure de méthylène, ajoute 70 cm3 d'acide trifluoroacétique et agite une heure 30 minutes à température ambiante. On concentre à sec sous pression réduite, reprend le résidu par du chlorure de méthylène et extrait avec une solution aqueuse à 10% de bicarbonate de sodium. On lave la phase aqueuse avec du chlorure de méthylène et neutralise avec de l'acide chlorhydrique concentré. On extrait au chlorure de méthylène, lave à l'eau salée, sèche et concentre à sec et obtient 5,3 g de produit attendu.
Rf. = 0,5 dans CH₂Cl₂ - MeOH - AcOH (9 - 0,5 - 0,5).

### Stade D : 2-(difluorométhyl) 2-(formylamino) 4-méthyl 4-penténoate d'éthyle.

On agite 10,5 g de produit obtenu ci-dessus dans 60 cm3 de chlorure de thionyle et chauffe au reflux pendant 3 heures. On évapore à sec, reprend le résidu dans le toluène et sèche sous pression réduite et obtient 10,5 g de chlorure d'acide.

On dissout ce dernier dans 50 cm3 d'acétone, refroidit à 0°C, ajoute goutte à goutte, une solution de 3,35 g d'azoture de sodium dans 20 cm3 d'eau et agite pendant une heure à 0°C. On évapore l'acétone, extrait à l'éther, lave à l'eau salée, sèche, évapore à sec et obtient 10 g d'azoture.

On dissout ce dernier dans 100 cm3 d'acide formique et chauffe au reflux pendant une heure trente minutes. On laisse revenir à température ambiante et ajoute goutte à goutte 40 cm3 d'anhydride acétique et agite 3 heures à température ambiante. On ajoute lentement 40 cm3 d'eau glacée, évapore à sec et reprend dans un mélange eau - chlorure de méthylène, extrait au chlorure de méthylène, lave avec une solution aqueuse à 10% de bicarbonate de sodium, puis à l'eau salée, sèche et concentre à sec. On chromatographie le résidu sur silice, élue par un mélange cyclohexane - acétate d'éthyle (85-15) et isole 5 g de produit attendu.

### Stade E : 2-(fluorométhyl) 2-(formylamino) 6-hydroxy 4-méthylène heptanedioate de diéthyle.

On met en suspension 9,65 g de chlorure ferrique dans 50 cm3 de chlorure de méthylène, ajoute goutte à goutte à goutte 3,03 g de glyoxylate d'éthyle dans 50 cm3 de chlorure de méthylène et agite 30 minutes à température ambiante. On refroidit à -60°C et ajoute goutte à goutte 3,5 g de produit obtenu en D dans 50 cm3 de chlorure de méthylène.

On agite une heure à -30°C puis une heure à -20°C. On verse le mélange réactionnel sur de l'eau glacée, extrait au chlorure de méthylène, lave à l'eau salée puis avec une solution aqueuse à 10% de bicarbonate de sodium puis à l'eau salée. On sèche et évapore à sec. On chromatographie sur silice le résidu, élue par un mélange cyclohexane - acétate d'éthyle (6-4) et isole 0,590 g de produit B (4-méthyl heptène...) et 1,7 g de produit C (4-méthylène - heptane...) et 1 g de mélange.

| | | |
|---|---|---|
| Produit B | Rf. 0,32 | dans cyclohexane - acétate d'éthyle |
| Produit C | Rf. 0,35 | (1 - 1) |

### Stade F : 2-(difluorométhyl) 2-(formylamino) 4-méthylène 6-[(méthylsulfonyl) oxy] heptanedioate de diéthyle.

On dissout 1,65 g de produit C obtenu ci-dessus dans 20 cm3 de pyridine, refroidit à 0°C et ajoute 0,725 g de chlorure de méthanesulfonyle.

On agite 30 minutes à 0°C et 5 heures à température ambiante. On verse le mélange réactionnel sur de l'acide chlorhydrique 6N glacé, extrait au chlorure de méthylène, lave la phase organique avec de l'acide chlorhydrique 6N puis avec une solution aqueuse à 10% de bicarbonate de sodium enfin à l'eau salée. On sèche et évapore à sec. On chromatographie le résidue sur silice, élue par un mélange chlorure de méthylène - acétate d'éthyle (9-1) et isole 1,55 g de produit attendu.
Rf. = 0,45 dans CH₂Cl₂ - AcOEt (8-2).

### Stade G : 2-(difluorométhyl) 2-(formylamino) 4-méthylène heptane dioate de diéthyle.

On dissout 0,430 g de produit obtenu en F dans 20 cm3 de diméthoxyéthane, ajoute 2 cm3 d'eau, 0,770 g d'iodure de sodium et 0,670 g de zinc, agite et porte au reflux pendant 9 heures. On filtre, ajoute 25 cm3 d'eau au filtrat et extrait au chlorure de méthylène. On lave la phase organique à l'eau salée, sèche et évapore à sec. On obtient 300 mg de produit attendu.
Rf. = 0,5 dans CH₂Cl₂ - AcOEt (8-2).

### Stade H : acide 2-amino 2(difluorométhyl) 4-méthylène heptane dioïque.

On dissout 250 mg de produit obtenu ci-dessus dans 5 cm3 d'éthanol, ajoute 0,5 cm3 d'acide chlorhydrique concentré, agite et chauffe au reflux pendant une heure. On neutralise avec du bicarbonate de soude et évapore à sec. On reprend dans l'eau et extrait à l'acétate d'éthyle, lave à l'eau salée, sèche et évapore à sec. On reprend le résidu dans l'éthanol, ajoute 2,5 cm3 de soude 0,1N et agite 16 heures à température ambiante. On neutralise à pH 6 avec de l'acide acétique, amène à sec, reprend dans 2 cm3 d'eau et passe sur résine échangeuse d'ions Dowex 50 W x 8, élue à l'eau puis à l'ammoniaque 0,7N. On obtient 130 mg de produit qu'on lyophilise dans 10 cm3 d'eau. On récupère 125 mg de produit attendu.
Rf. = 0,6 dans BuOH - AcOH - H₂O (4-2-2).

| Spectre RMN (ppm) | |
|---|---|
| 6,28 | CHF |
| 2,56 et 2,92 | CH₂ en position 3 |
| 5,06 et 5,11 | CH₂ du méthylène substitué en position 4. |

### Exemple 13 : acide 2-(L-alanylamino)3-heptènedioïque.

On met en suspension 1,83 g de N-[(1,1-diméthyl éthoxy) carbonyl] L-alaninate de (2,5-dioxopyrrolidin-1-yle) dans 10 cm3 de diméthyl formamide, refroidit à 0°C et ajoute goutte à goutte 0,250 g d'acide 2-amino 3-heptènedioïque dans 20 cm3 de diméthylformamide en maintenant le pH entre 8 et 9 à l'aide de triéthylamine et agite 16 heures à température ambiante. On concentre à sec, reprend par 10 cm3 d'eau, lave avec de l'acétate d'éthyle, amène à pH 1 avec de l'acide chlorhydrique 2N et extrait à l'acétate d'éthyle, sèche les phases organiques. On chromatographie le résidu sur silice, élue par un mélange : chlorure de méthylène - méthanol - acide formique (95-5-1) et isole 0,467 g de produit bloqué.

On reprend 0,400 g de ce dernier dans 10 cm3 d'acide trifluoroacétique dans le chlorure de méthylène (1-1) et laisse en contact pendant une heure à température ambiante. On amène à sec le milieu réactionnel, reprend le résidu deux fois par du chlorure de méthylène et amène à sec, reprend le résidu à l'eau et ajuste le pH 7 avec de la soude 2N.

On passe cette solution sur résine échangeuse d'ions Dowex 50 W x 8, élue à l'ammoniaque 0,2M et obtient 0,211 g de produit que l'on purifie sur silice éluants : éthanol - ammoniaque (9-1) puis (8-2).

On isole 108 mg de produit attendu après lyophilisation.

### Préparation de N-[(1,1-diméthyléthoxy) carbonyl] L-alaninate de (2,5-dioxopyrrolidin-1-yle).

On dissout 0,2 g de B.O.C. L-alanine et 0,122 g de 1-hydroxy 2,5-dioxopyrrolidine dans 5 cm3 de diméthoxyéthane. On refroidit à 0°C et ajoute peu à peu 0,24 g de dicyclohexyl carbodiimide et laisse en contact pendant 7 heures. On filtre, amène à sec, reprend par du chlorure de méthylène, lave avec une solution aqueuse à 10% de bicarbonate de sodium, sèche et obtient 0,305 g de produit brut. On recristallise celui-ci dans 1,5 cm3 d'isopropanol et obtient 0,220 g de produit attendu. F = 164°C.

### Exemple 14 : Acide 6-[[N-(1-oxo octadécyl) gamma-D-glutamyl] amino] 3-heptènedioïque (sel de sodium)

### Stade A : 6-[[N-(1-oxo octadécyl) gamma-D-glutamyl] amino] 3-heptènedioate d'éthyle

On refroidit à 14°C, 1,28 g d'acide 4-[N-1-oxo octadécyl] glutamique en solution dans un mélange d'oxanne/tétrahydrofuranne (3-1) puis ajoute en 5 minutes 0,86 cm³ de triéthylamine puis 0,43 cm³ de chloroformiate d'isobutyle.

### Exemple 15 : Acide 6-[(gamma-D-glutamyl) amino] 3-heptènedioïque

### Stade A : 6-[(N-trifluoroacétyl gamma-D-glutamyl) amino] 3-heptènedioate d'éthyle.

On ajoute en 15 minutes 554 mg d'acide glutamique en solution dans 30 cm³ de diméthoxyéthane dans 422 mg du produit obtenu comme au stade D de l'exemple 5. On refroidit la solution à 0°C, ajoute en 10 minutes 475 mg de dicyclohexylcarbodiimide en solution dans 10 cm³ de diméthoxy éthane et agite la suspension obtenue 16 heures à 0°C puis 4 heures à température ambiante. On filtre et évapore à sec. On reprend le résidu huileux dans 10 cm³ d'éther, filtre et évapore à sec. On récupère 1,03 g de produit brut que l'on purifie par chromatographie sur silice (éluant cyclohexane - acétate d'éthyle 5-5). On obtient 410 mg de produit attendu.

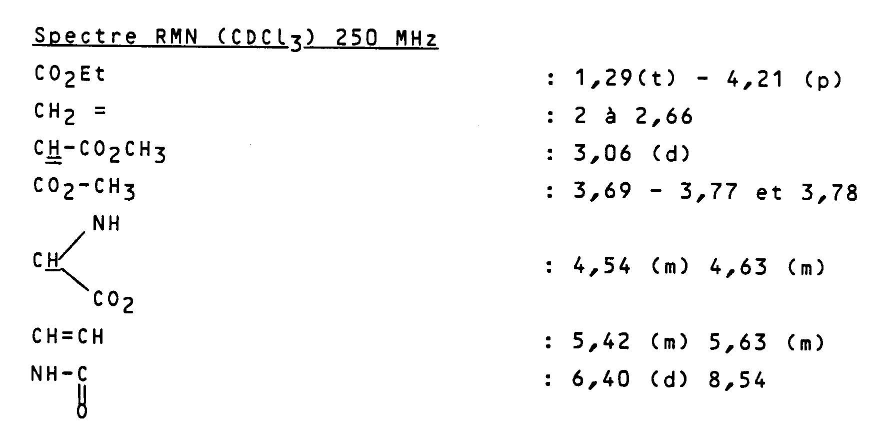

### Stade B : Acide 6-[(gamma-D-glutamyl) amino] 3-heptènedioïque

On dissout 454 mg de produit préparé comme au stade A dans 20 cm³ d'éthanol, refroidit à 0°C et ajoute 3,6 cm³ de soude N. On agite, ajoute 1 cm³ d'eau et maintient 16 heures sous agitation à température ambiante. On concentre sous pression réduite, reprend le résidu dans 2 cm³ d'eau et amène à pH 6 à l'aide d'une solution d'acide chlorhydrique 2N. On purifie par passage sur résine Dowex (50 W x 8) en éluant à l'eau puis à l'ammoniaque. On obtient 272 mg de produit que l'on dissout dans 10 cm³ puis lyophilise. On recueille 215 mg de produit attendu.
alpha_{D} = -18° ± 2° (c = 0,5 % HCl₆N)

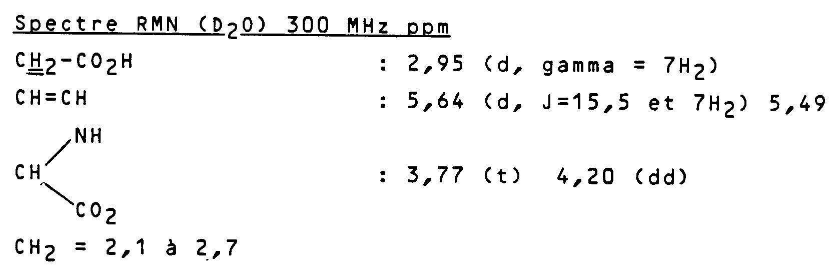

### Exemple 16 :

On a préparé des comprimés répondant à la formule :

| | |
|---|---|
| Produit de l'exemple 6 | 50 mg |
| Excipient q.s. pour un comprimé terminé à | 250 mg. |
| (Détail de l'excipient : lactose, amidon, talc, stéarate de magnésium). | |

### Exemple 17 :

On a préparé des comprimés répondant à la formule :

| | |
|---|---|
| Produit de l'exemple 2 | |
| Excipient q.s pour un comprimé terminé à | 250 mg |
| (Détail de l'excipient : lactose, amidon, talc, stéarate de magnésium). | |

### ETUDE PHARMACOLOGIQUE

### Stimulation des cellules monocytaires par un immunostimulant.

Les cellules mononuclées du sang circulant de donneurs normaux sont séparées suivant le technique classique décrite par Boyum utilisant un gradient de Ficoll. Après lavage, les mononucléaires sont mis à incuber à 37°C pendant une heure à raison de 5.10⁶ monocytes (cellules NSE⁺) par ml de milieu de culture, 5 ml par flacon de culture. Le milieu de culture utilisé dans cette expérience se compose de RPMI 1640 additionné d'antibiotiques et de tampon Hépès. Au bout d'une heure, les cellules non adhérentes sont enlevées par lavage des flacons à l'aide de milieu précédemment porté à 37°C, les cellules adhérentes se composant essentiellement de monocytes (>90%) sont remises en culture dans du milieu RPMI complet sans sérum en présence de différentes quantités de produits à tester précédemment mis en solution dans un tampon (Dulbecco) sans Ca²⁺ ni Mg²⁺. La culture est poursuivie pendant 24 ou 48 heures et les surnageants des cellules sont alors prélevés, centrifugés, aliquotés et conservés soit à -80°C soit à -20°C. Le surnageants de culture sont remplacés, dans les flacons par la même quantité d'eau distillée apyrogène afin de lyser les cellules. Le lysat est récupéré, aliquoté et conservé également à -20°C. Les expériences suivantes ont été réalisées en présence ou en l'absence d'Interferon gamma (10³ U/ml) à une dose où l'IFN gamma seul est inactif.

### Tests de la présence dans les surnageants de monokines (Interleukine-1 et Tumor Necrosis Factor) par leur activité biologique.

### Test Interleukine-1 (IL-1) :

Ce test a été décrit la première fois par I. Gery et Waksman en 1972 (Gery I and B.H. Waksman 1972. Potentiation of the T lymphocyte response to mitogens. II. The cellular source of potentiating mediator (s) J.Exp.Med., 136-143.
Il est basé sur l'action co-mitogène de l'IL-1 en présence d'un antigène (mimé par la phytohémaglutinine dans le test) sur des thymocytes de souris. 1,5 x 10⁶ thymocytes de souris C3H/HeJ (provenant du C.S.E.A.L. d'Orléans) sont mis en culture pendant trois jours en présence de différentes dilutions de surnageants et de lysats cellulaires susceptibles de contenir de l'activité IL-1 et de PHA-P Wellcome (1 µg/ml) dans des plaques de culture 96 puits fond plat, dans un volume final de 200 µl de milieu composé de RPMI 1640 contenant outre des antibiotiques (pénicilline 1 U/ml - steptomycine 1000 U/ml du tampon Hépès 1 mM de la glutamine, 2mM 5% de sérum de veau et du 2-mercaptoéthanol 5 x 10⁻⁵M. Au bout de 68 heures de culture, 1 µCi de thymidine tritiée est ajoutée à chaque puits (³H-méthyl-thymidine, CEA Saclay, TMM79A activité spécifique 1 Ci/mM = 37GBq/mM), la radioactivité incorporée par les cellules est évaluée après avoir filtré les cultures sur appareil collecteur semi-automatique de type Skatron et compté les filtres dans un compteur à scintillation liquide (Beckman). Les résultats sont exprimés par la différence entre les impulsions par minutes incorporées par les cultures en présence de surnageants et les impulsions par minutes incorporées par les cultures témoins.

### Test Tumor Necrosis Factor (TNF) :

L'activité TNF est mise en évidence par la toxicité de ce facteur sur des cellules cibles L-929 (sous-clone alpha). La technique a été sensibilisée en ajoutant dans le test de l'actinomycine D.

Les cellules L sont distribuées à raison de 2 x10⁴ cellules par puits d'une microplaque à fond plat dans 100 µl de milieu RPMI 1640 enrichi avec 5% de sérum de veau, de la glutamine, du tampon Hépès et des antibiotiques. Au bout de 24 heures, différentes dilutions des surnageants à tester sont ajoutés dans un volume de 100 ul ainsi qu'une dose d'actinomycine D de 1 µg/ml. Au bout de 24 heures de culture, la quantité de cellules non lysées est évaluée en colorant les plaques au violet crystal et en mesurant la densité optique des différents puits sur lecteur multiscan.

### RESULTATS :

Le produit de l' exemple 9 stimule les monocytes et leur production d'IL-1 et de TNF. De plus, il y a synergie entre les produits et l'Interféron gamma.

### B. Activité antibactérienne (in vitro)

L'activité antibactérienne des produits revendiqués a été déterminée par la méthode de diffusion en milieu de Davis Mingioli additionné de 1% d'agar. Les géloses utilisées sont coulées en boîtes de Petri à 48°C, après ensemencement à 5x10⁻⁵ germes/ml au moyen de la souche bactérienne test. Les inocula proviennent d'une pré-culture de 24 heures en bouillon de Davis Mingioli. Après durcissement des géloses, les solutions aqueuses des produits étudiés sont introduites dans des puits (9 mm) creusés dans le milieu à l'aide d'un emporte-pièce. Les zones d'inhibition observées (diamètre en mm) sont mesurées après incubation de 24 heures à 37°C.

| | Produit de l'exemple 6 (25 mg/l) | Produit de l'exemple 13 (25 mg/l) |
|---|---|---|
| Escherichia Coli 078 | | 17,5 |
| Salmonella typhimurium MZ11 | 24 | 20 |
| Enterobacter cloacae 1321E | 10 | |
| Providencia sp. DU48 | | 22 |

### C. Activité antibactérienne (in vivo)

Des souris Swiss femelles pesant de 18 à 20 g (Charles River Cobs non consanguines CD1) ont été utilisées pour cette expérimentation. Chaque lot est composé de 20 animaux.
Les différents produits sont mis en solution dans du sérum physiologique apyrogène de facon à obtenir des concentrations de 1 - 10 - 100 mcg/souris soit : 50 - 500 - 5000 mcg/kg.

### Préparation de la suspension infectante (souche IP 52 145)

### A) Conservation et entretien de la souche

La souche lyophilisée est réhydratée par quelques gouttes de bouillon trypcase soja (BioMérieux). Un tube de 10 ml de bouillon trypcase soja est ensemencé avec la suspension obtenue et cultivé 18 heures à l'étuve à 37°C.
Des tubes de gélose de conservation (Pasteur) sont ensemencés avec cette culture par piqure centrale. Après 18 heures de culture à l'étuve à 37°C, les tubes sont conservés à 22°C.

### B) Mise en culture

Un tube de bouillon trypcase soja est ensemencé à partir d'une gélose de conservation et mis à cultiver 18 heures à l'étuve à 37°C. Des géloses inclinées trypcase soja sont ensemencées à partir de cette culture.
Après 18 heures d'étuve à 37°C les germes sont récupérés à la surface de la gélose et mis en suspension dans du sérum physiologique à raison d'une anse de platine pour 10 ml d'eau physiologique.
La concentration en germes est évaluée par comptage au Coulter Counter. La suspension bactérienne est diluée jusqu'à l'obtention de la dose de germes désirée puis injectée par voie I.V. sous un volume de 0,2 ml/souris. La quantité de germes revivifiables injectés est contrôlée par étalement sur boîtes de Petri contenant de la gélose trypcase.

### Protocole.

L'administration a été faite par une injection par voie intramusculaire sous un volume de 0,05 ml/souris 24 heures avant l'infection.

Les germes sont injectés par voie intraveineuse sous un volume de 0,2 ml/souris. Les animaux sont observés pendant 21 jours. Une comparaison de la mortalité des animaux non traités (témoin) est effectuée par rapport à celle des animaux traités par les différents produits.

Les résultats sont exprimés soit en survie (nombre d'animaux survivants dans chaque groupe au terme des 21 jours d'observation). Le test de Fisher est utilisé en comparant les survies des groupes traités au groupe témoin. Dans le cas où on ne peut détecter une augmentation nette de la survie après 21 jours d'observation, on peut calculer pour chacun des groupes un temps moyen de survie (TMS). Dans ce cas, l'analyse statistique est faite par le test de Mann and Whitney en comparant chacun des groupes traités au groupe témoin. L'étude a été faite sur ordinateur IBM PC et logiciel Tadpole. Une protection particulièrement significative est observée dans le groupe traité par le produit de l'exemple 8 à la dose de 5 mg/kg : résultats exprimés en TMS ou en survie au terme des 21 jours d'observation.

### D. ACTIVITE SUR LES MONOCYTES HUMAINS

Les produits des exemples 5 et 9 ont montré une action directe sur les monocytes humains, action mesurée par la production d'une activité TNF et d'une activité IL-1 dans les surnageants de culture (avec 5 g de produit).

De plus, avec le produit de l'exemple 9, en présence de CHU-IFN-g, on a pu mettre en évidence un effet au moins additif dans la production de monokines (TNF et IL-1).

## Revendications (Revendications pour l'(les) Etat(s) contractant(s) suivant(s): AT, BE, CH, DE, FR, GB, IT, LI, LU, NL, SE)

1. les composés de formule (I) : 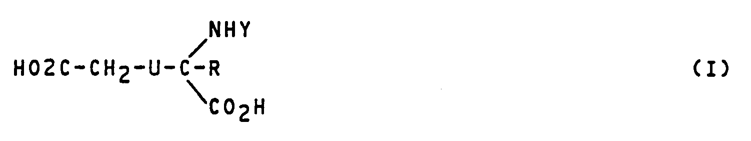 dans laquelle U représente un radical 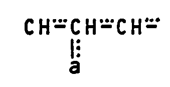 dans lequel les traits pointillés représentent une seule double liaison de configuration cis ou trans et a représente un atome d'hydrogène, un radical méthyle ou un groupement méthylène -CH₂-, Y représente un atome d'hydrogène ou le reste d'un acide aminé lié par le carboxyle alpha ou oméga ou d'un peptide constitué de 2, 3 ou 4 acides aminés dont l'amine est éventuellement acylé par un acide aliphatique saturé ou insaturé renfermant de 6 à 24 atomes de carbone et R représente un atome d'hydrogène ou un radical alkyle, linéaire ou ramifié, saturé ou insaturé, renfermant jusqu'à 8 atomes de carbone éventuellement substitué par un ou plusieurs atomes d'halogène, à l'exception de l'acide trans 3,4-déhydro-D-2-aminopimélique ainsi que leurs esters avec les alcools renfermant jusqu'à 6 atomes de carbone, leurs amides primaires et secondaires et leurs sels avec les acides organiques ou minéraux ou avec les bases.

2. Les composés de formule I tels que définis à la revendication 1 dans lesquels U représente un radical :
-CH=CH-CH₂-
ou un radical
-CH₂-CH=CH-
ainsi que leurs esters avec les alcools renfermant jusqu'à 6 atomes de carbone, leurs amides primaires et secondaires et leurs sels avec les acides organiques ou minéraux ou avec les bases.

3. Les composés de formule (I) tels que définis à la revendication 1 et 2 dans lesquels Y est choisi dans le groupe des restes de l'alanine, la proline, la lysine et l'acide alpha ou gamma glutamique ainsi que leurs esters avec les alcools renfermant jusqu'a 6 atomes de carbone, leurs amides primaires et secondaires et leurs sels avec les acides organiques ou minéraux ou avec les bases.

4. Les composés de formule (I) tels que définis à la revendication 3 dans lesquels Y représente le reste de l'alanine ainsi que leurs esters avec les alcools renfermant jusqu'à 6 atomes de carbone, leurs amides primaires et secondaires et leurs sels avec les acides organiques ou minéraux ou avec les bases.

5. Les composés de formule (I) tels que définis à la revendication 3 dans lesquels Y représente le reste de la lysine ainsi que leurs esters avec les alcools renfermant jusqu'à 6 atomes de carbone, leurs amides primaires et secondaires et leurs sels avec les acides organiques ou minéraux ou avec les bases.

6. Les composés de formule (I) tels que définis à l'une quelconque des revendications 1 à 5 dans lesquels R représente un atome d'hydrogène ainsi que leurs esters avec les alcools renfermant jusqu'à 6 atomes de carbone, leurs amides primaires et secondaires et leurs sels avec les acides organiques ou minéraux ou avec les bases.

7. Les composés de formule (I) tels que définis à l'une quelconque les revendications 1 à 5 dans lesquels R représente un radical méthyle ainsi que leurs esters avec les alcools renfermant jusqu'à 6 atomes de carbone, leurs amides primaires et secondaires et leurs sels avec les acides organiques ou minéraux ou avec les bases.

8. Les composés de formule (I) tels que définis à l'une quelconque des revendications 1 à 5 dans lesquels R représente un radical CHF₂ ou encore éthynyle ou vinyle ainsi que leurs esters avec les alcools renfermant jusqu'à 6 atomes de carbone, leurs amides primaires et secondaires et leurs sels avec les acides organiques ou minéraux ou avec les bases.

9. Les composés de formule (I) tels que définis à la revendication 1 dont le nom suit :
l'acide 6-(L-lysylamino) 3-heptènedioïque ainsi que ses esters avec les alcools renfermant jusqu'à 6 atomes de carbone, leurs amides primaires et secondaires et leurs sels avec les bases.

10. Les composés de formule (I) tels que définis à la revendication 1 dont les noms suivent :
- l'acide 6-[L-alanylamino] 3-heptènedioïque,
- l'acide 2-[L-alanylamino] 3-heptènedioïque,
ainsi que leurs esters avec les alcools, renfermant jusqu'à 6 atomes de carbone, leurs amides primaires et secondaires et leurs sels avec les bases.

11. A titre de médicaments, les composés de formule (I) tels que définis à l'une quelconque des revendications 1 à 8.

12. A titre de médicaments, les composés de formule (I) tels que définis à la revendication 9 ou 10.

13. Les compositions pharmaceutiques renfermant comme principe actif au moins l'un des médicaments définis aux revendications 11 et 12.

14. Procédé de préparation des produits de formule (I) tels que définis à l'une quelconque des revendications 1 à 10, caractérisé en ce que l'on soumet un composé de formule (II) : 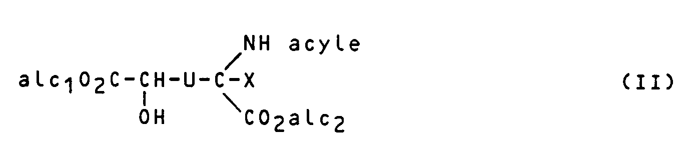 dans laquelle U conserve sa signification précédente, alc₁ et alc₂, identiques ou différents, représentent un radical alkyle renfermant jusqu'à 8 atomes de carbone, X représente soit le radical R soit un groupement -CO₂alc₃ dans lequel alc₃ représente un radical alkyle renfermant jusqu'à 8 atomes de carbone et acyle représente le reste d'un acide organique renfermant jusqu'à 8 atomes de carbone, à l'action d'un halogénure d'alcanesulfonyle ou d'aryl sulfonyle de formule (III) :
HalSO₂alc₄ (III)
dans lequel alc₄ représente un radical alkyle renfermant jusqu'à 8 atomes de carbone ou un radical aryle renfermant jusqu'à 14 atomes de carbone, puis à l'action d'un agent de réduction pour obtenir le composé de formule (IV) : 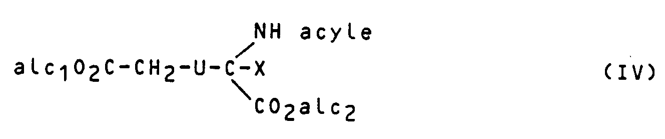 que l'on soumet, si désiré, à la totalité ou à une partie seulement des opérations suivantes dans un ordre quelconque :
a) déprotection de la fonction amine,
b) hydrolyse des fonctions esters, puis le cas échéant décarboxylation lorsque X représente un radical -CO₂alc₃,
c) réduction de la double liaison,
d) estérification ou salification par une base des fonctions carboxy,
e) salification des fonctions amino par un acide,
f) amidification des fonctions aminées libres par un acide aminé ou un peptide constitué de 2, 3 ou 4 acides aminés dont la fonction aminée est protégée puis déprotection de cette fonction aminée.

15. Procédé selon la revendication 14, caractérisé en ce que l'on effectue sur le dérivé de formule : 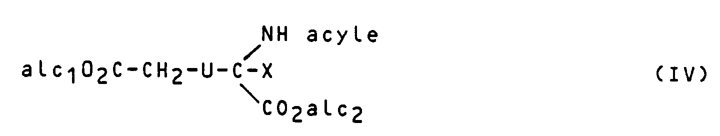 dans laquelle alc₁, alc₂, acyle et X sont définis comme dans la revendication 14, les opérations suivantes et dans cet ordre :
a) déprotection de la fonction aminée,
b) hydrolyse des fonctions esters,
pour obtenir le composé de formule (I_{A}) : 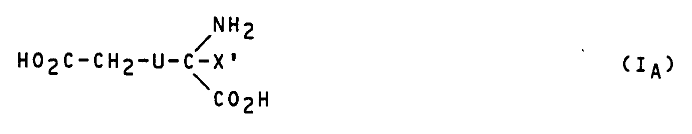 dans laquelle X′ représente soit le radical R soit un groupement -CO₂H, produit I_{A} que, si désiré, on l'amidifie par un acide aminé ou un peptide constitué de 2, 3 ou 4 acides aminés dont la fonction aminée est protégée, puis soumet le cas échéant à un réactif de décarboxylation la fonction X′, puis déprotège la fonction amino du produit obtenu que l'on réduit, si désiré.

16. Variante du procédé de la revendication 14, caractérisée en ce que l'on soumet le composé de formule : 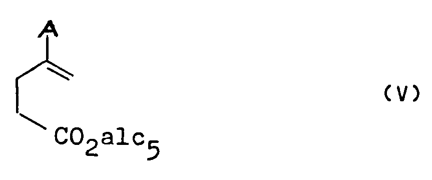 dans laquelle alc₅ représente un radical alkyle renfermant jusqu'à 8 atomes de carbone et A représente un atome d'hydrogène ou un radical méthyle, à l'action d'un composé de formule (VI) : 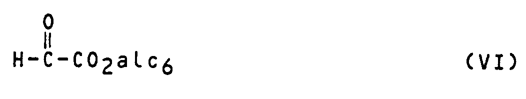 dans laquelle alc₆ représente un radical alkyle renfermant jusqu'à 8 atomes de carbone, pour obtenir le composé de formule (VII) : 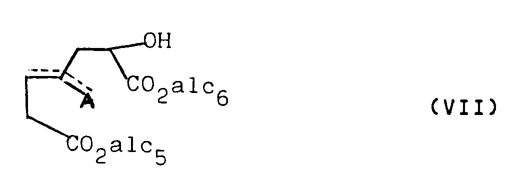 que l'on soumet à l'action d'un halogénure d'alcanesulfonyle ou d'arylsulfonyle de formule (VIII) :
HalSO₂alc₇ (VIII)
dans lequel alc₇ représente un radical alkyle renfermant jusqu'à 3 atomes ou un radical aryle renfermant jusqu'à 14 atomes de carbone, pour obtenir le composé de formule (IX) : 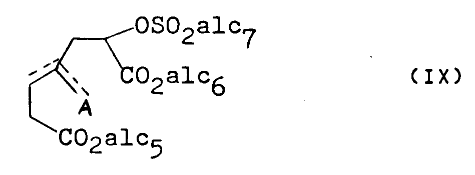 que l'on soumet, à l'action d'un azoture alcalin, pour obtenir le composé de formule (X) : 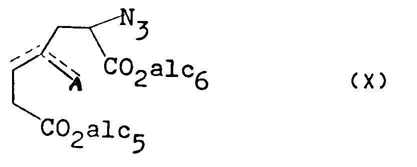 que l'on réduit et soumet le produit ainsi obtenu à une hydrolyse aqueuse, pour obtenir le composé de formule (XI) : 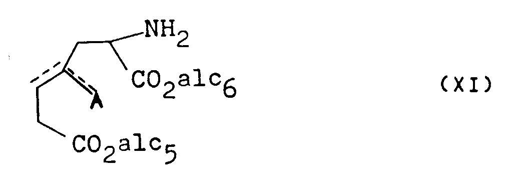 que l'on soumet, si désiré, à la totalité ou à seulement une partie des opérations suivantes dans un ordre quelconque :
a) hydrolyse des fonctions esters,
b) amidification des fonctions amino libres par un acide aminé ou un peptide constitué de 2, 3 ou 4 acides aminés dont la fonction amino est protégée puis déprotection de cette fonction amino,
c) réduction de la double liaison,
d) estérification ou salification par une base des fonctions carboxy,
e) salification par un acide des fonctions amino.

## Revendications (Revendications pour l'(les) Etat(s) contractant(s) suivant(s): ES, GR)

1. Procédé de préparation des composés de formule (I) : 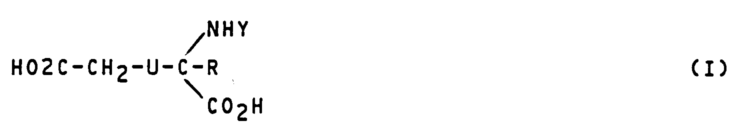 dans laquelle U représente un radical 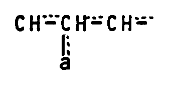 dans lequel les traits pointillés représentent une seule double liaison de configuration cis ou trans et a représente un atome d'hydrogène, un radical méthyle ou un groupement méthylène -CH₂-, Y représente un atome d'hydrogène ou le reste d'un acide aminé lié par le carboxyle alpha ou oméga ou d'un peptide constitué de 2, 3 ou 4 acides aminés dont l'amine est éventuellement acylé par un acide aliphatique saturé ou insaturé renfermant de 6 à 24 atomes de carbone et R représente un atome d'hydrogène ou un radical alkyle, linéaire ou ramifié, saturé ou insaturé, renfermant jusqu'à 8 atomes de carbone éventuellement substitué par un ou plusieurs atomes d'halogène, à l'exception de l'acide trans 3,4-déhydro-D-2-aminopimélique ainsi que de leurs esters avec les alcools renfermant jusqu'à 6 atomes de carbone, leurs amides primaires et secondaires et leurs sels avec les acides organiques ou minéraux ou avec les bases, caractérisé en ce que l'on soumet un composé de formule (II) : 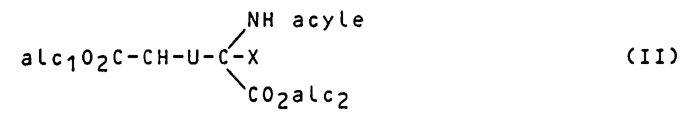 dans laquelle U conserve sa signification précédente, alc₁ et alc₂, identiques ou différents, représentent un radical alkyle renfermant jusqu'à 8 atomes de carbone, X représente soit le radical R soit un groupement -CO₂alc₃ dans lequel alc₃ représente un radical alkyle renfermant jusqu'à 8 atomes de carbone et acyle représente le reste d'un acide organique renfermant jusqu'à 8 atomes de carbone, à l'action d'un halogénure d'alcanesulfonyle ou d'aryl sulfonyle de formule (III) :
HalSO₂alc₄ (III)
dans lequel alc₄ représente un radical alkyle renfermant jusqu'à 8 atomes de carbone ou un radical aryle renfermant jusqu'à 14 atomes de carbone, puis à l'action d'un agent de réduction pour obtenir le composé de formule (IV) : 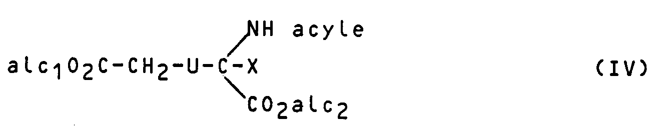 que l'on soumet, si désiré, à la totalité ou à une partie seulement des opérations suivantes dans un ordre quelconque :
a) déprotection de la fonction amine,
b) hydrolyse des fonctions esters, puis le cas échéant décarboxylation lorsque X représente un radical -CO₂alc₃,
c) réduction de la double liaison,
d) estérification ou salification par une base des fonctions carboxy,
e) salification des fonctions amino par un acide,
f) amidification des fonctions aminées libres par un acide aminé ou un peptide constitué de 2, 3 ou 4 acides aminés dont la fonction aminée est protégée puis déprotection de cette fonction aminée.

2. Procédé selon la revendication 1, caractérisé en ce que l'on effectue sur le dérivé de formule : 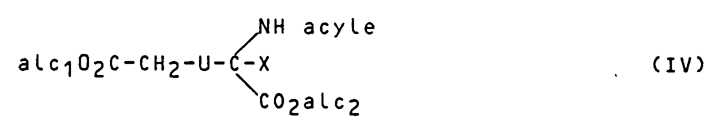 dans laquelle alc₁, alc₂, acyle et X sont définis comme dans la revendication 14, les opérations suivantes et dans cet ordre :
a) déprotection de la fonction aminée,
b) hydrolyse des fonctions esters,
pour obtenir le composé de formule (I_{A}) : 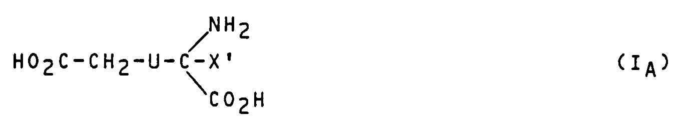 dans laquelle X' représente soit le radical R soit un groupement -CO₂H, produit I_{A} que, si désiré, on l'amidifie par un acide aminé ou un peptide constitué de 2, 3 ou 4 acides aminés dont la fonction aminée est protégée, puis soumet le cas échéant à un réactif de décarboxylation la fonction X', puis déprotège la fonction amino du produit obtenu que l'on réduit, si désiré.

3. Variante du procédé de la revendication 1, caractérisée en ce que l'on soumet le composé de formule : 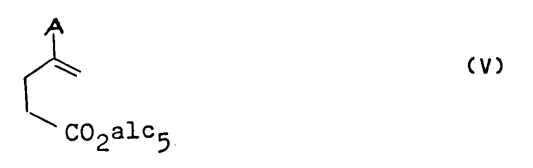 dans laquelle alc₅ représente un radical alkyle renfermant jusqu'à 8 atomes de carbone et A représente un atome d'hydrogène ou un radical méthyle, à l'action d'un composé de formule (VI) : 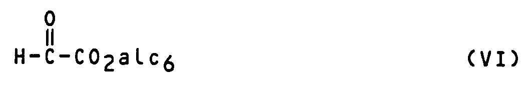 dans laquelle alc₆ représente un radical alkyle renfermant jusqu'à 8 atomes de carbone, pour obtenir le composé de formule (VII) : 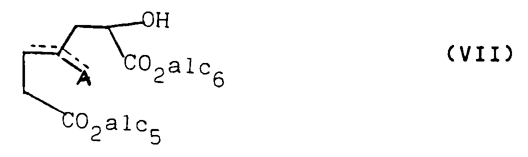 que l'on soumet à l'action d'un halogénure d'alcanesulfonyle ou d'arylsulfonyle de formule (VIII) :
HalSO₂alc₇ (VIII)
dans lequel alc₇ représente un radical alkyle renfermant jusqu'à 3 atomes ou un radical aryle renfermant jusqu'à 14 atomes de carbone, pour obtenir le composé de formule (IX) : 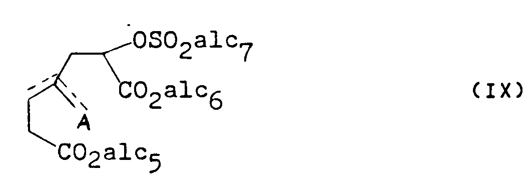 que l'on soumet, à l'action d'un azoture alcalin, pour obtenir le composé de formule (X) : 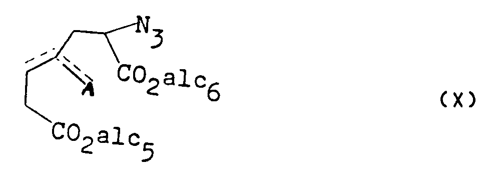 que l'on réduit et soumet le produit ainsi obtenu à une hydrolyse aqueuse, pour obtenir le composé de formule (XI) : 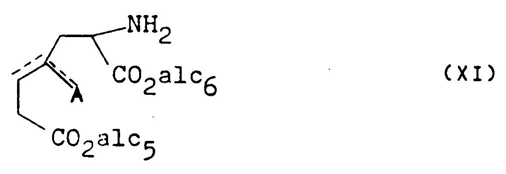 que l'on soumet, si désiré, à la totalité ou à seulement une partie des opérations suivantes dans un ordre quelconque :
a) hydrolyse des fonctions esters,
b) amidification des fonctions amino libres par un acide aminé ou un peptide constitué de 2, 3 ou 4 acides aminés dont la fonction amino est protégé puis déprotection de cette fonction amino,
c) réduction de la double liaison,
d) estérification ou salification par une base des fonctions carboxy,
e) salification par un acide des fonctions amino.

4. Procédé selon la revendication 1 ou 2 caractérisé en ce que l'on utilise au départ un composé de formule II dans laquelle U représente un radical -CH=CH-CH₂ ou un radical -CH₂-CH=CH-.

5. Procédé selon l'une des revendications 1 à 4 pour préparer des produits de formule I dans laquelle Y est choisi dans le groupe des restes de l'alanine, la proline, la lysine et l'acide alpha ou gamma glutamique ainsi que leurs esters avec les alcools renfermant jusqu'à 6 atomes de carbone, leurs amides primaires et secondaires et leurs sels avec les acides organiques ou minéraux ou avec les bases, caractérisé en ce que l'on amidifie de façon appropriée la fonction amine libre du produit de formule XI ou celle du produit obtenu après déprotection de la fonction -NH acyle du produit de formule IV.

6. Procédé selon l'une des revendications 1 à 4 pour préparer des produits de formule I dans laquelle Y représente le reste de l'alanine ainsi que leurs esters avec les alcools renfermant jusqu'à 6 atomes de carbone, leurs amides primaires et secondaires et leurs sels avec les acides organiques ou minéraux ou avec les bases caractérisé en ce que l'on amidifie de facon appropriée la fonction amine libre du produit de formule XI ou celle du produit obtenu après déprotection de la fonction -NH acyle du produit de formule IV.

7. Procédé selon l'une des revendications 1 à 4 pour préparer des produits de formule I dans laquelle Y représente le reste de la lysine ainsi que leurs esters avec les alcools renfermant jusqu'à 6 atomes de carbone, leurs amides primaires et secondaires et leurs sels avec les acides organiques ou minéraux ou avec les bases caractérisé en ce que l'on amidifie de facon appropriée la fonction amine libre du produit de formule XI ou celle du produit obtenu après déprotection de la fonction -NH acyle du produit de formule IV.

8. Procédé selon l'une des revendications 1 à 3 pour préparer des produits de formule I dans lesquels R représente un atome d'hydrogène ainsi que leurs esters avec les alcools renfermant jusqu'à 6 atomes de carbone, leurs amides primaires et secondaires et leurs sels avec les acides organiques ou minéraux ou avec les bases, caractérisé en ce que l'on utilise au départ un produit de formule II dans laquelle X représente un atome d'hydrogène ou soumet le produit de formule IV à un réactif approprié de décarboxylation.

9. Procédé selon l'une des revendications 1 à 3 pour préparer des produits de formule I dans lesquels R représente un radical méthyle ainsi que leurs esters avec les alcools renfermant jusqu'à 6 atomes de carbone, leurs amides primaires et secondaires et leurs sels avec les acides organiques ou minéraux ou avec les bases, caractérisé en ce que l'on utilise au départ un produit de formule II dans laquelle X représente un radical méthyle.

10. Procédé selon l'une des revendications 1 à 3 pour préparer des produits de formule I dans lesquels R représente un radical CHF₂ ou encore éthynyle ou vinyle ainsi que leurs esters avec les alcools renfermant jusqu'à 6 atomes de carbone, leurs amides primaires et secondaires et leurs sels avec les acides organiques ou minéraux ou avec les bases caractérisé en ce que l'on utilise au départ un produit de formule II dans laquelle X représente un radical CHF₂ ou encore éthynyl ou vinyl.

11. Procédé selon la revendication 1 caractérisé en ce que l'on choisit le produit de départ de manière telle que l'on prépare l'acide 6-(L-lysylamino) 3-heptènedioïque ainsi que ses esters avec les alcools renfermant jusqu'à 6 atomes de carbone, leurs amides primaires et secondaires et leurs sels avec les bases.

12. Procédé selon la revendication 1 caractérisé en ce que l'on choisit le produit de départ de manière telle que l'on prépare :
- l'acide 6-[L-alanylamino] 3-heptènedioïque,
- l'acide 2-[L-alanylamino] 3-heptènedioïque,
ainsi que leurs esters avec les alcools, renfermant jusqu'à 6 atomes de carbone, leurs amides primaires et secondaires et leurs sels avec les bases.

## Claims (Claims for the following Contracting State(s): AT, BE, CH, DE, FR, GB, IT, LI, LU, NL, SE)

1. The compounds of formula (I): 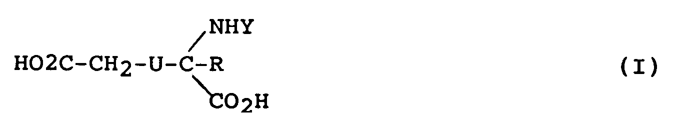 in which U represents a 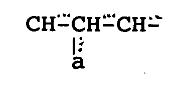 radical in which the dotted lines represent a single double bond of cis or trans configuration and a represents a hydrogen atom, a methyl radical or a methylene -CH₂- group, Y represents a hydrogen atom or the remainder of an amino acid linked by the alpha or omega carboxyl or of a peptide constituted by 2, 3 or 4 amino acids the amine of which is optionally acylated by a saturated or unsaturated aliphatic acid containing 6 to 24 carbon atoms and R represents a hydrogen atom or a saturated or unsaturated, linear or branched alkyl radical containing up to 8 carbon atoms optionally substituted by one or more halogen atoms, with the exception of trans 3,4-dehydro-D-2-aminopimelic acid, as well as their esters with alcohols containing up to 6 carbon atoms, their primary and secondary amides and their salts with organic or mineral acid or with bases.

2. The compounds of formula (I) as defined in claim 1 in which U represents a radical:
-CH=CH-CH₂-
or a radical
-CH₂-CH=CH-
as well as their esters with alcohols containing up to 6 carbon atoms, their primary and secondary amides and their salts with organic or mineral acids or with bases.

3. The compounds of formula (I) as defined in claim 1 and 2 in which Y is chosen from the group of remainders of alanine, proline, lysine and alpha or gamma glutamic acid as well as their esters with alcohols containing up to 6 carbon atoms, their primary and secondary amides and their salts with organic or mineral acids or with bases.

4. The compounds of formula (I) as defined in claim 3 in which Y represents the remainder of alanine as well as their esters with alcohols containing up to 6 carbon atoms, their primary and secondary amides and their salts with organic or mineral acids or with bases.

5. The compounds of formula (I) as defined in claim 3 in which Y represents the remainder of lysine as well as their esters with alcohols containing up to 6 carbon atoms, their primary and secondary amides and their salts with organic or mineral acids or with bases.

6. The compounds of formula (I) as defined in any one of claims 1 to 5 in which R represents a hydrogen atom as well as their esters with alcohols containing up to 6 carbon atoms, their primary and secondary amides and their salts with organic or mineral acids or with bases.

7. The compounds of formula (I) as defined in any one of claims 1 to 5 in which R represents a methyl radical as well as their esters with alcohols containing up to 6 carbon atoms, their primary and secondary amides and their salts with organic or mineral acids or with bases.

8. The compounds of formula (I) as defined in any one of claims 1 to 5 in which R represents a CHF₂ radical or also ethynyl or vinyl as well as their esters with alcohols containing up to 6 carbon atoms, their primary or secondary amides and their salts with organic or mineral acids or with bases.

9. The compounds of formula (I) as defined in claim 1 of which the name follows:
6-(L-lysylamino)-3-heptenedioic acid as well as its esters with alcohols containing up to 6 carbon atoms, their primary and secondary amides and their salts with bases.

10. The compounds of formula (I) as defined in claim 1 of which the names follow:
- 6-[L-alanylamino]-3-heptenedioic acid,
- 2-[L-alanylamino]-3-heptenedioic acid,
as well as their esters with alcohols, containing up to 6 carbon atoms, their primary and secondary amides and their salts with bases.

11. As medicaments, the compounds of formula (I) as defined in any one of claims 1 to 8.

12. As medicaments, the compounds of formula (I) as defined in claim 9 or 10.

13. The pharmaceutical compositions containing as active ingredient at least one of the medicaments defined in claims 11 and 12.

14. Preparation process for products of formula (I) as defined in any one of claims 1 to 10, characterized in that a compound of formula (II): 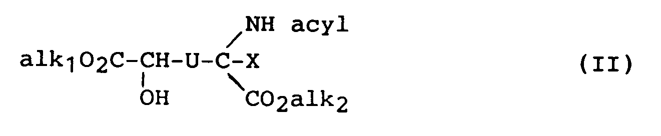 in which U keeps its previous meaning, alk₁ and alk₂, identical or different, represent an alkyl radical containing up to 8 carbon atoms, X represents either the R radical or a -CO₂alk₃ group in which alk₃ represents an alkyl radical containing up to 8 carbon atoms and acyl represents the remainder of an organic acid containing up to 8 carbon atoms, is subjected to the action of an alkanesulphonyl or arylsulphonyl halide of formula (III):
HalSO₂alk₄ (III)
in which alk₄ represents an alkyl radical containing up to 8 carbon atoms or an aryl radical containing up to 14 carbon atoms, then to the action of a reducing agent in order to obtain the compound of formula (IV): 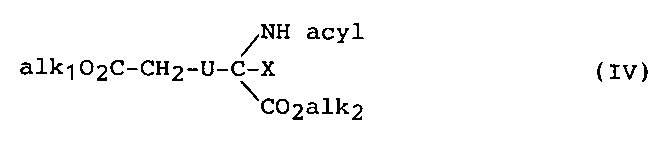 which is subjected, if desired, to all or a part only of the following operations in any order:
a) deprotection of the amine function,
b) hydrolysis of the ester functions, then if appropriate decarboxylation when X represents a -CO₂alk₃ radical,
c) reduction of the double bond,
d) esterification or salification of the carboxy functions by a base,
e) salification of the amino functions by an acid,
f) amidification of the free amino functions by an amino acid or a peptide constituted by 2, 3 or 4 amino acids the amino function of which is protected then deprotection of this amino function.

15. Process according to claim 14, characterized in that on a derivative of formula: 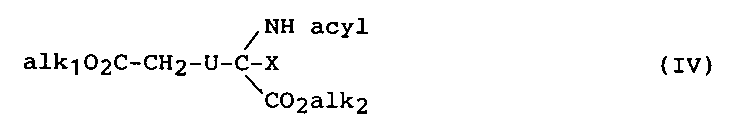 in which alk₁, alk₂, acyl and X are defined as in claim 14, the following operations are carried out in this order:
a) deprotection of the amino function,
b) hydrolysis of the ester functions,
in order to obtain the compound of formula (I_{A}) 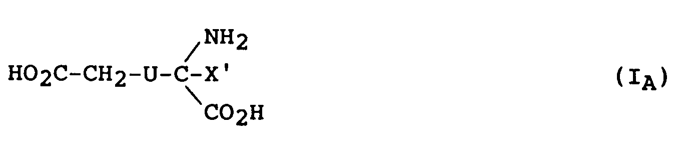 in which X' represents either the R radical or a -CO₂H group, which product I_{A} is, if desired, amidified by an amino acid or a peptide constituted by 2, 3 or 4 amino acids the amino function of which is protected, then if appropriate the X' function is subjected to a decarboxylation reagent, then the amino function of the product obtained is deprotected and, if desired, reduced.

16. Variant of the process of claim 14, characterized in that the compound of formula: 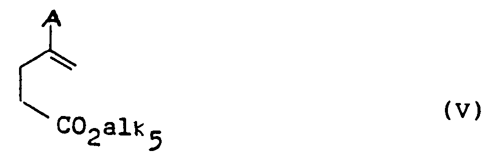 in which alk₅ represents an alkyl radical containing up to 8 carbon atoms and A represents a hydrogen atom or a methyl radical, is subjected to the action of a compound of formula (VI): 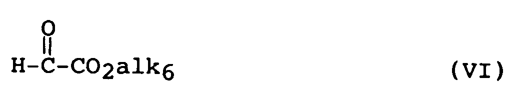 in which alk₆ represents an alkyl radical containing up to 8 carbon atoms, in order to obtain the compound of formula (VII): 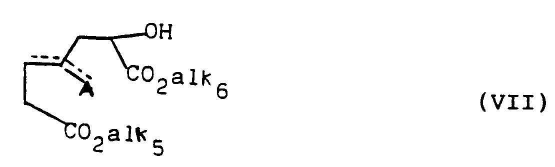 which is subjected to the action of an alkanesulphonyl or arylsulphonyl halide of formula (VIII):
HalSO₂alk₇ (VIII)
in which alk₇ represents an alkyl radical containing up to 3 atoms or an aryl radical containing up to 14 carbon atoms, in order to obtain the compound of formula (IX): 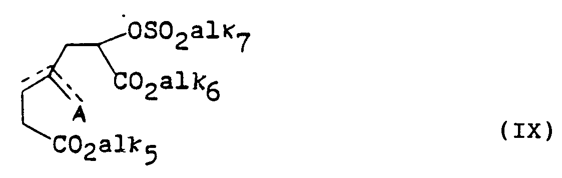 which is subjected to the action of an alkaline nitride, in order to obtain the compound of formula (X): 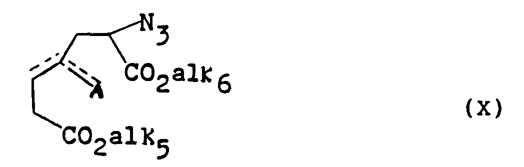 which is reduced and the product thus obtained is subjected to an aqueous hydrolysis, in order to obtain the compound of formula (XI): 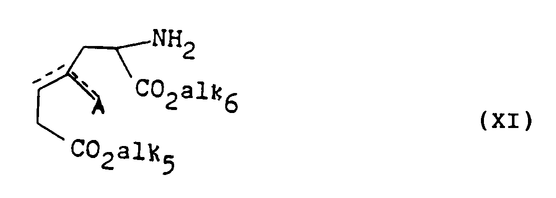 which is subjected, if desired, to all or a part only of the following operations in any order:
a) hydrolysis of the ester functions,
b) amidification of the free amino functions by an amino acid or a peptide constituted by 2, 3 or 4 amino acids the amino function of which is protected then deprotection of this amino function,
c) reduction of the double bond,
d) esterification or salification of the carboxy functions by a base,
e) salification of the amino functions by an acid.

## Claims (Claims for the following Contracting State(s): ES, GR)

1. Preparation process for compounds of formula (I): 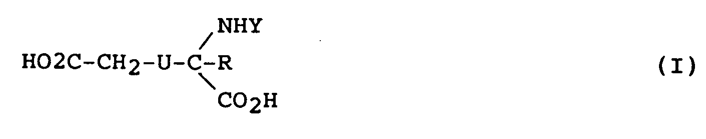 in which U represents a 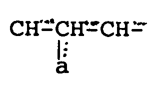 radical in which the dotted lines represent a single double bond of cis or trans configuration and a represents a hydrogen atom, a methyl radical or a methylene -CH₂- group, Y represents a hydrogen atom or the remainder of an amino acid linked by the alpha or omega carboxyl or of a peptide constituted by 2, 3 or 4 amino acids the amine of which is optionally acylated by a saturated or unsaturated aliphatic acid containing 6 to 24 carbon atoms and R represents a hydrogen atom or a saturated or unsaturated, linear or branched alkyl radical containing up to 8 carbon atoms optionally substituted by one or more halogen atoms, with the exception of trans 3,4-dehydro-D-2-aminopimelic acid, as well as their esters with alcohols containing up to 6 carbon atoms, their primary and secondary amides and their salts with organic or mineral acids or with bases, characterized in that a compound of formula (II): 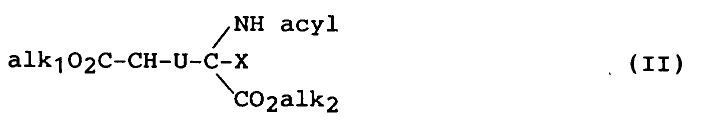 in which U keeps its previous meaning, alk₁ and alk₂, identical or different, represent an alkyl radical containing up to 8 carbon atoms, X represents either the R radical or a -CO₂alk₃ group in which alk₃ represents an alkyl radical containing up to 8 carbon atoms and acyl represents the remainder of an organic acid containing up to 8 carbon atoms, is subjected to the action of an alkanesulphonyl or arylsulphonyl halide of formula (III):
HalSO₂alk₄ (III)
in which alk₄ represents an alkyl radical containing up to 8 carbon atoms or an aryl radical containing up to 14 carbon atoms, then to the action of a reducing agent in order to obtain the compound of formula (IV): 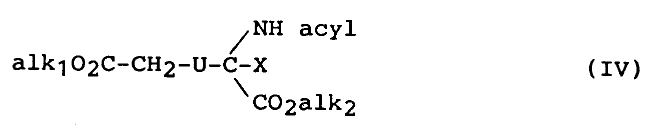 which is subjected, if desired, to all or a part only of the following operations in any order:
a) deprotection of the amine function,
b) hydrolysis of the ester functions, then if appropriate decarboxylation when X represents a -CO₂alk₃ radical,
c) reduction of the double bond,
d) esterification or salification of the carboxy functions by a base,
e) salification of the amino functions by an acid,
f) amidification of the free amino functions by an amino acid or a peptide constituted by 2, 3 or 4 amino acids the amino function of which is protected then deprotection of this amino function.

2. Process according to claim 1, characterized in that on a derivative of formula: 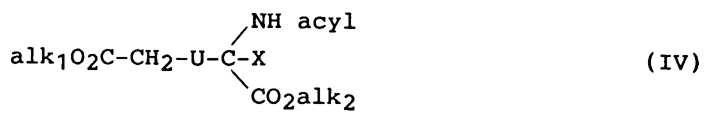 in which alk₁, alk₂, acyl and X are defined as in claim 14, the following operations are carried out in this order:
a) deprotection of the amino function,
b) hydrolysis of the ester functions,
in order to obtain the compound of formula (I_{A}) 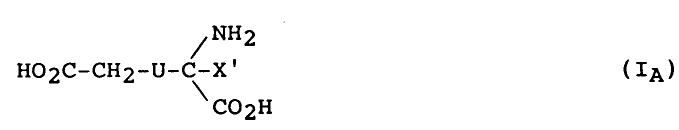 in which X' represents either the R radical or a -CO₂H group, which product I_{A} is, if desired, amidified by an amino acid or a peptide constituted by 2, 3 or 4 amino acids the amino function of which is protected, then if appropriate the X' function is subjected to a decarboxylation reagent, then the amino function of the product obtained is deprotected and, if desired, reduced.

3. Variant of the process of claim 1, characterized in that the compound of formula: 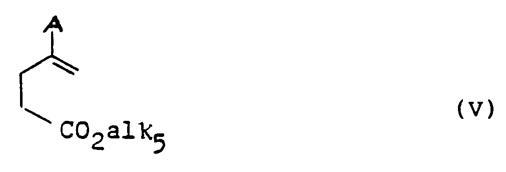 in which alk₅ represents an alkyl radical containing up to 8 carbon atoms and A represents a hydrogen atom or a methyl radical, is subjected to the action of a compound of formula (VI): 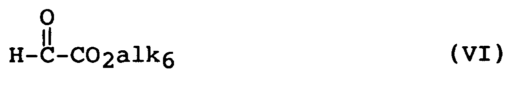 in which alk₆ represents an alkyl radical containing up to 8 carbon atoms, in order to obtain the compound of formula (VII): 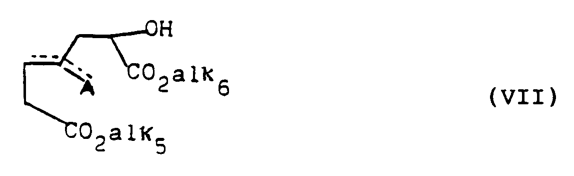 which is subjected to the action of an alkanesulphonyl or arylsulphonyl halide of formula (VIII):
HalSO₂alk₇ (VIII)
in which alk₇ represents an alkyl radical containing up to 3 atoms or an aryl radical containing up to 14 carbon atoms, in order to obtain the compound of formula (IX): 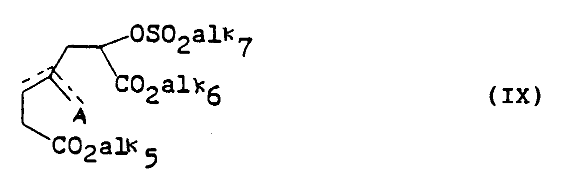 which is subjected to the action of an alkaline nitride, in order to obtain the compound of formula (X): 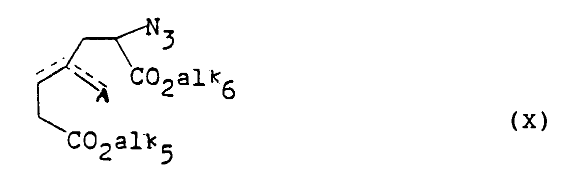 which is reduced and the product thus obtained is subjected to an aqueous hydrolysis, in order to obtain the compound of formula (XI): 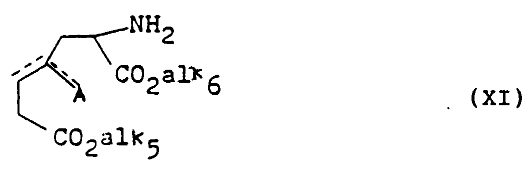 which is subjected, if desired, to all or a part only of the following operations in any order:
a) hydrolysis of the ester functions,
b) amidification of the free amino functions by an amino acid or a peptide constituted by 2, 3 or 4 amino acids the amino function of which is protected then deprotection of this amino function,
c) reduction of the double bond,
d) esterification or salification of the carboxy functions by a base,
e) salification of the amino functions by an acid.

4. Process according to claim 1 or 2, characterized in that a compound of formula (II) is used at the start in which U represents a -CH=CH-CH₂ radical or a -CH₂-CH=CH- radical.

5. Process according to one of claims 1 to 4 for preparing products of formula I in which Y is chosen from the group of remainders of alanine, proline, lysine and alpha or gamma glutamic acid as well as their esters with alcohols containing up to 6 carbon atoms, their primary and secondary amides and their salts with organic or mineral acids or with bases, characterized in that the free amine function of the product of formula XI or that of the product obtained after deprotection of the -NH acyl function of the product of formula IV is amidified in an appropriate way.

6. Process according to one of claims 1 to 4 for preparing products of formula I in which Y represents the remainder of alanine as well as their esters with alcohols containing up to 6 carbon atoms, their primary and secondary amides and their salts with organic or mineral acids or with bases, characterized in that the free amine function of the product of formula XI or that of the product obtained after deprotection of the -NH acyl function of the product of formula IV is amidified in an appropriate way.

7. Process according to one of claims 1 to 4 for preparing products of formula I in which Y represents the remainder of lysine as well as their esters with alcohols containing up to 6 carbon atoms, their primary and secondary amides and their salts with organic or mineral acids or with bases, characterized in that the free amine function of the product of formula XI or that of the product obtained after deprotection of the -NH acyl function of the product of formula IV is amidified in an appropriate way.

8. Process according to one of claims 1 to 3 for preparing products of formula I in which R represents a hydrogen atom as well as their esters with alcohols containing up to 6 carbon atoms, their primary and secondary amides and their salts with organic or mineral acids or with bases, characterized in that a product of formula II is used at the start in which X represents a hydrogen atom or the product of formula IV is subjected to an appropriate decarboxylation reagent.

9. Process according to one of claims 1 to 3 for preparing products of formula I in which R represents a methyl radical as well as their esters with alcohols containing up to 6 carbon atoms, their primary and secondary amides and their salts with organic or mineral acids or with bases, characterized in that a product of formula II is used at the start in which X represents a methyl radical.

10. Process according to one of claims 1 to 3 for preparing products of formula I in which R represents a CHF₂ radical or also ethynyl or vinyl as well as their esters with alcohols containing up to 6 carbon atoms, their primary and secondary amides and their salts with organic or mineral acids or with bases, characterized in that a product of formula II is used at the start in which X represents a CHF₂ radical or also ethynyl or vinyl.

11. Process according to claim 1 characterized in that the starting product is chosen in such a way that 6-(L-lysylamino)-3-heptenedioic acid is prepared, as well as its esters with alcohols containing up to 6 carbon atoms, their primary and secondary amides and their salts with bases.

12. Process according to claim 1 characterized in that the starting product is chosen in such a way that:
- 6-[L-alanylamino]-3-heptenedioic acid,
- 2-[L-alanylamino]-3-heptenedioic acid,
are prepared, as well as their esters with alcohols, containing up to 6 carbon atoms, their primary and secondary amides and their salts with bases.

## Patentansprüche (Patentansprüche für folgende(n) Vertragsstaat(en): AT, BE, CH, DE, FR, GB, IT, LI, LU, NL, SE)

1. Verbindungen der Formel (I) 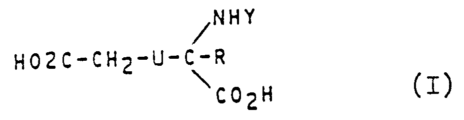 worin U für einen Rest 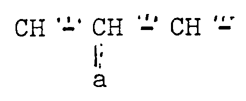 steht, in dem die gestrichelten Linien eine einzige Doppelbindung mit cis- oder trans-Konfiguration wiedergeben, und a ein Wasserstoffatom, einen Methylrest oder eine Methylengruppe -CH₂- bedeutet, Y ein Wasserstoffatom oder den Rest einer über das α- oder ω-Carboxyl gebundenen Aminosäure oder eines aus 2, 3 oder 4 Aminosäuren bestehenden Peptids bedeutet, deren Amin gegebenenfalls durch eine gesättigte oder ungesättigte, aliphatische Säure mit 6 bis 24 Kohlenstoffatomen acyliert ist, und R für ein Wasserstoffatom oder einen gesättigten oder ungesättigten, linearen oder verzweigten Alkylrest mit bis zu 8 Kohlenstoffatomen, der gegebenenfalls durch ein oder mehrere Halogenatome substituiert ist, steht, mit Ausnahme der trans-3,4-Dehydro-D-2-aminopimelinsäure sowie deren Ester mit Alkoholen mit bis zu 6 Kohlenstoffatomen, deren primäre und sekundäre Amide und deren Salze mit organischen oder Mineralsäuren oder mit Basen.

2. Verbindungen der Formel I, wie in Anspruch 1 definiert, worin U einen Rest
-CH=CH-CH₂-
oder einen Rest
-CH₂-CH=CH-
bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

3. Verbindungen der Formel (I), wie in Anspruch 1 und 2 definiert, worin Y unter den Resten von Alanin, Prolin, Lysin und der α- oder γ-Glutaminsäure ausgewählt ist, sowie deren Ester mit bis zu 6 Kohlenstoffatome enthaltenden Alkoholen, deren primäre und sekundäre Amide und deren Salze mit organischen oder Mineralsäuren oder mit Basen.

4. Verbindungen der Formel (I), wie in Anspruch 3 definiert, worin Y den Alaninrest bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

5. Verbindungen der Formel (I), wie in Anspruch 3 definiert, worin Y den Lysinrest bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

6. Verbindungen der Formel (I), wie in einem der Ansprüche 1 bis 5 definiert, worin R ein Wasserstoffatom bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

7. Verbindungen der Formel (I), wie in einem der Ansprüche 1 bis 5 definiert, worin R einen Methylrest bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

8. Verbindungen der Formel (I), wie in einem der Ansprüche 1 bis 5 definiert, worin R einen Rest CHF₂ oder auch Ethinyl oder Vinyl bedeutet, sowie deren Ester mit bis zu 6 Kohlenstoffatome enthaltenden Alkoholen, deren primäre und sekundäre Amide und deren Salze mit organischen oder Mineralsäuren oder mit Basen.

9. Verbindungen der Formel (I), wie in Anspruch 1 definiert, mit der folgenden Bezeichnung:
6-(L-Lysylamino)-3-heptendisäure sowie ihre Ester mit bis zu 6 Kohlenstoffatome enthaltenden Alkoholen, deren primäre und sekundäre Amide und deren Salze mit Basen.

10. Verbindungen der Formel (I), wie in Anspruch 1 definiert, mit den folgenden Bezeichnungen:
6-(L-Alanylamino)-3-heptendisäure,
2-(L-Alanylamino)-3-heptendisäure
sowie deren Ester mit bis zu 6 Kohlenstoffatome enthaltenden Alkoholen, deren primäre und sekundäre Amide und deren Salze mit Basen.

11. Als Arzneimittel die Verbindungen der Formel (I), wie in einem der Ansprüche 1 bis 8 definiert.

12. Als Arzneimittel die Verbindungen der Formel (I), wie in Anspruch 9 oder 10 definiert.

13. Pharmazeutische Zusammensetzungen, enthaltend als Wirkstoff zumindest eines der Arzneimittel, wie in den Ansprüchen 11 und 12 definiert.

14. Verfahren zur Herstellung der Produkte der Formel (I), wie in einem der Ansprüche 1 bis 10 definiert, dadurch gekennzeichnet, daß man eine Verbindung der Formel (II) 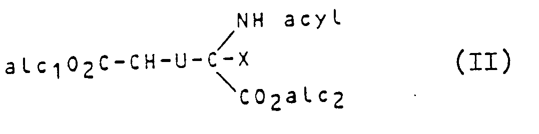 worin U die vorstehend angegebene Bedeutung besitzt, alc₁ und alc₂, die gleich oder voneinander verschieden sind, einen Alkylrest mit bis zu 8 Kohlenstoffatomen bedeuten, X entweder den Rest R oder eine Gruppe -CO₂alc₃, worin alc₃ einen Alkylrest mit bis zu 8 Kohlenstoffatomen wiedergibt, bedeutet und acyl den Rest einer organischen Säure mit bis zu 8 Kohlenstoffatomen wiedergibt, der Einwirkung eines Alkansulfonyl- oder Arylsulfonylhalogenids der Formel (III)
HalSO₂alc₄ (III)
worin alc₄ für einen Alkylrest mit bis zu 8 Kohlenstoffatomen oder einen Arylrest mit bis zu 14 Kohlenstoffatomen steht, danach der Einwirkung eines Reduktionsmittels unterzieht, um die Verbindung der Formel (IV) 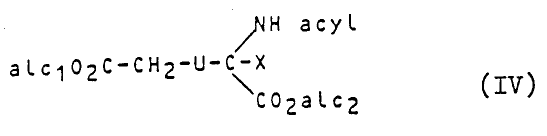 zu erhalten, welche man gewünschtenfalls sämtlichen oder lediglich einem Teil der folgenden Maßnahmen in beliebiger Reihenfolge unterzieht:
(a) Abspaltung der Schutzgruppe von der Aminfunktion,
(b) Hydrolyse der Esterfunktionen, danach gegebenenfalls Decarboxylierung, wenn X einen Rest -CO₂alc₃ bedeutet,
(c) Reduktion der Doppelbindung,
(d) Veresterung oder Salzbildung mit einer Base der Carboxyfunktionen,
(e) Salzbildung der Aminofunktionen durch eine Säure,
(f) Amidierung der freien Aminofunktionen mit einer Aminosäure oder einem aus 2, 3 oder 4 Aminosäuren bestehenden Peptid, deren Aminfunktion geschützt ist, danach Abspaltung der Schutzgruppe von dieser Aminfunktion.

15. Verfahren gemäß Anspruch 14, dadurch gekennzeichnet, daß man an dem Derivat der Formel 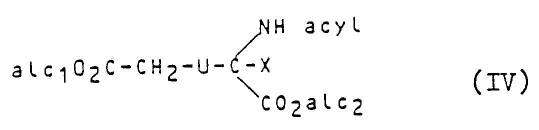 worin alc₁, alc₂, acyl und X die in Anspruch 14 angegebene Bedeutung besitzen, die folgenden Maßnahmen in der angegebenen Reihenfolge durchführt:
(a) Schutzgruppen-Abspaltung von der Aminfunktion,
(b) Hydrolyse der Esterfunktionen zur Erzielung der Verbindung der Formel (I_{A}) 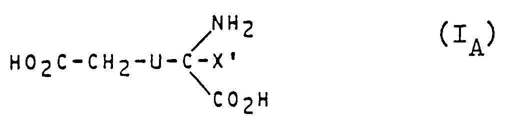
worin X' entweder den Rest R oder eine Gruppe -CO₂H wiedergibt, welches Produkt I_{A} man gewünschtenfalls mit einer Aminosäure oder einem aus 2, 3 oder 4 Aminosäuren bestehenden Peptid, deren Aminfunktion geschützt ist, amidiert, danach gegebenenfalls die Funktion X' einem Decarboxylierungsreagens unterzieht, wonach man die Schutzgruppe von der Aminofunktion des erhaltenen Produkts, welches man gewünschtenfalls reduziert, abspaltet.

16. Abänderung des Verfahrens gemäß Anspruch 14, dadurch gekennzeichnet, daß man die Verbindung der Formel 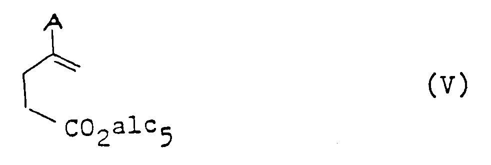 worin alc₅ einen Alkylrest mit bis zu 8 Kohlenstoffatomen bedeutet und A für ein Wasserstoffatom oder eine Methylgruppe steht, der Einwirkung einer Verbindung der Formel (VI) 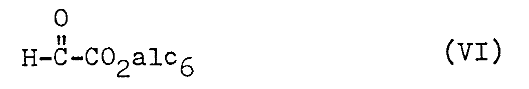 worin alc₆ einen Alkylrest mit bis zu 8 Kohlenstoffatomen bedeutet, unterzieht, um die Verbindung der Formel (VII) 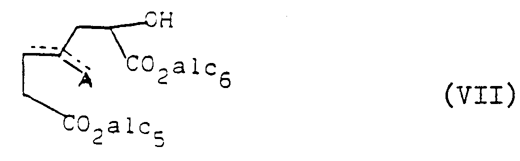 zu erhalten, welche man der Einwirkung eines Alkansulfonyl- oder Arylsulfonylhalogenids der Formel (VIII)
HalSO₂alc₇ (VIII)
worin alc₇ einen Alkylrest mit bis zu 3 Kohlenstoffatomen oder einen Arylrest mit bis zu 14 Kohlenstoffatomen bedeutet, unterzieht, um die Verbindung der Formel (IX) 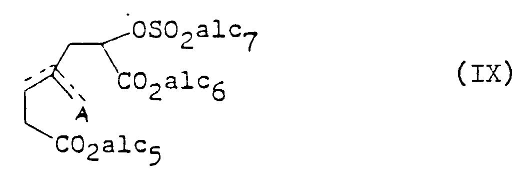 zu erhalten, welche man der Einwirkung eines Alkalinitrids unterzieht, um die Verbindung der Formel (X) 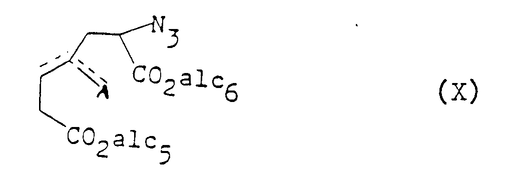 zu erhalten, welche man reduziert und das so erhaltene Produkt einer wäßrigen Hydrolyse unterwirft, um zu der Verbindung der Formel (XI) 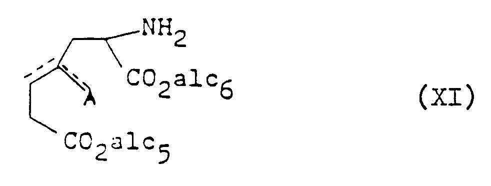 zu gelangen, welche man gewünschtenfalls sämtlichen oder lediglich einem Teil der folgenden Maßnahmen in beliebiger Reihenfolge unterzieht:
(a) Hydrolyse der Esterfunktionen,
(b) Amidierung der freien Aminofunktionen mit einer Aminosäure oder einem aus 2, 3 oder 4 Aminosäuren bestehenden Peptid, deren Aminofunktion geschützt ist, danach Abspaltung der Schutzgruppe von dieser Aminofunktion,
(c) Reduktion der Doppelbindung,
(d) Veresterung oder Salzbildung mit einer Base der Carboxyfunktionen,
(e) Salzbildung mit einer Säure der Aminofunktionen.

## Patentansprüche (Patentansprüche für folgende(n) Vertragsstaat(en): ES, GR)

1. Verfahren zur Herstellung der Verbindungen der Formel (I) 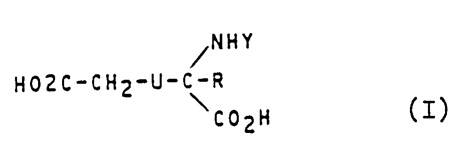 worin U für einen Rest 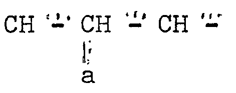 steht, in dem die gestrichelten Linien eine einzige Doppelbindung mit cis- oder trans-Konfiguration wiedergeben, und a ein Wasserstoffatom, einen Methylrest oder eine Methylengruppe -CH₂- bedeutet, Y ein Wasserstoffatom oder den Rest einer über das α- oder ω-Carboxyl gebundenen Aminosäure oder eines aus 2, 3 oder 4 Aminosäuren bestehenden Peptids bedeutet, deren Amin gegebenenfalls durch eine gesättigte oder ungesättigte, aliphatische Säure mit 6 bis 24 Kohlenstoffatomen acyliert ist, und R für ein Wasserstoffatom oder einen gesättigten oder ungesättigten, linearen oder verzweigten Alkylrest mit bis zu 8 Kohlenstoffatomen, der gegebenenfalls durch ein oder mehrere Halogenatome substituiert ist, steht, mit Ausnahme der trans-3,4-Dehydro-D-2-aminopimelinsäure sowie von deren Estern mit Alkoholen mit bis zu 6 Kohlenstoffatomen, deren primären und sekundären Amiden und deren Salzen mit organischen oder Mineralsäuren oder mit Basen,
dadurch gekennzeichnet, daß man eine Verbindung der Formel (II) 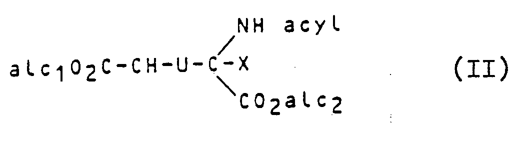 worin U die vorstehend angegebene Bedeutung besitzt, alc₁ und alc₂, die gleich oder voneinander verschieden sind, einen Alkylrest mit bis zu 8 Kohlenstoffatomen bedeuten, X entweder den Rest R oder eine Gruppe -CO₂alc₃, worin alc₃ einen Alkylrest mit bis zu 8 Kohlenstoffatomen wiedergibt, bedeutet und acyl den Rest einer organischen Säure mit bis zu 8 Kohlenstoffatomen wiedergibt, der Einwirkung eines Alkansulfonyl- oder Arylsulfonylhalogenids der Formel (III)
HalSO₂alc₄ (III)
worin alc₄ für einen Alkylrest mit bis zu 8 Kohlenstoffatomen oder einen Arylrest mit bis zu 14 Kohlenstoffatomen steht, danach der Einwirkung eines Reduktionsmittels unterzieht, um die Verbindung der Formel (IV) 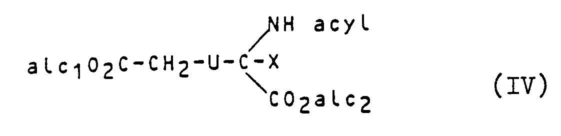 zu erhalten, welche man gewünschtenfalls sämtlichen oder lediglich einem Teil der folgenden Maßnahmen in beliebiger Reihenfolge unterzieht:
(a) Abspaltung der Schutzgruppe von der Aminfunktion,
(b) Hydrolyse der Esterfunktionen, danach gegebenenfalls Decarboxylierung, wenn X einen Rest -CO₂alc₃ bedeutet,
(c) Reduktion der Doppelbindung,
(d) Veresterung oder Salzbildung mit einer Base der Carboxyfunktionen,
(e) Salzbildung der Aminofunktionen durch eine Säure,
(f) Amidierung der freien Aminofunktionen mit einer Aminosäure oder einem aus 2, 3 oder 4 Aminosäuren bestehenden Peptid, deren Aminfunktion geschützt ist, danach Abspaltung der Schutzgruppe von dieser Aminfunktion.

2. Verfahren gemäß Anspruch 1, dadurch gekennzeichnet, daß man an dem Derivat der Formel 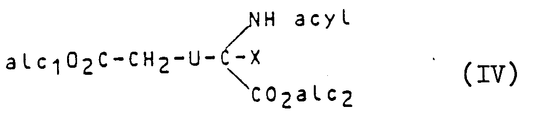 worin alc₁, alc₂, acyl und X die in Anspruch 1 angegebene Bedeutung besitzen, die folgenden Maßnahmen in der angegebenen Reihenfolge durchführt:
(a) Schutzgruppen-Abspaltung von der Aminfunktion,
(b) Hydrolyse der Esterfunktionen zur Erzielung der Verbindung der Formel (I_{A}) 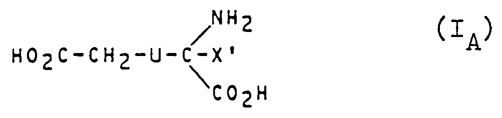
worin X' entweder den Rest R oder eine Gruppe -CO₂H wiedergibt, welches Produkt I_{A} man gewünschtenfalls mit einer Aminosäure oder einem aus 2, 3 oder 4 Aminosäuren bestehenden Peptid, deren Aminfunktion geschützt ist, amidiert, danach gegebenenfalls einem Decarboxylierungsreagens für die Funktion X' unterzieht, wonach man die Schutzgruppe von der Aminofunktion des erhaltenen Produkts, welches man gewünschtenfalls reduziert, abspaltet.

3. Abänderung des Verfahrens gemäß Anspruch 1, dadurch gekennzeichnet, daß man die Verbindung der Formel 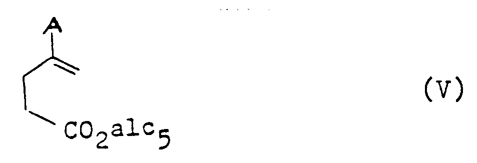 worin alc₅ einen Alkylrest mit bis zu 8 Kohlenstoffatomen bedeutet und A für ein Wasserstoffatom oder eine Methylgruppe steht, der Einwirkung einer Verbindung der Formel (VI) 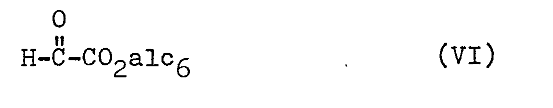 worin alc₆ einen Alkylrest mit bis zu 8 Kohlenstoffatomen bedeutet, unterzieht, um die Verbindung der Formel (VII) 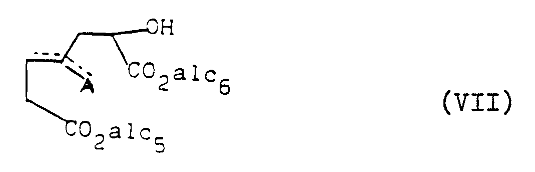 zu erhalten, welche man der Einwirkung eines Alkansulfonyl- oder Arylsulfonylhalogenids der Formel (VIII)
HalSO₂alc₇ (VIII)
worin alc₇ einen Alkylrest mit bis zu 3 Kohlenstoffatomen oder einen Arylrest mit bis zu 14 Kohlenstoffatomen bedeutet, unterzieht, um die Verbindung der Formel (IX) 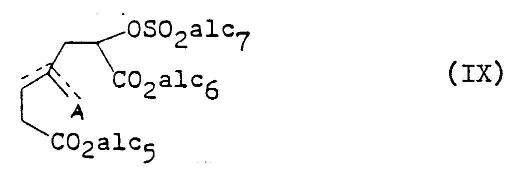 zu erhalten, welche man der Einwirkung eines Alkalinitrids unterzieht, um die Verbindung der Formel (X) 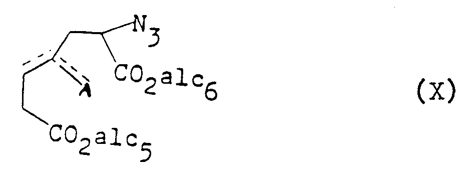 zu erhalten, welche man reduziert und das so erhaltene Produkt einer wäßrigen Hydrolyse unterwirft, um zu der Verbindung der Formel (XI) 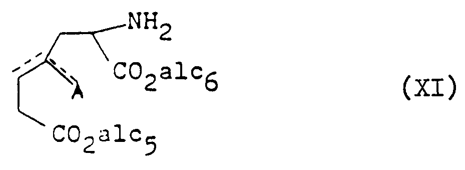 zu gelangen, welche man gewünschtenfalls sämtlichen oder lediglich einem Teil der folgenden Maßnahmen in beliebiger Reihenfolge unterzieht:
(a) Hydrolyse der Esterfunktionen,
(b) Amidierung der freien Aminofunktionen mit einer Aminosäure oder einem aus 2, 3 oder 4 Aminosäuren bestehenden Peptid, deren Aminofunktion geschützt ist, danach Abspaltung der Schutzgruppe von dieser Aminofunktion,
(c) Reduktion der Doppelbindung,
(d) Veresterung oder Salzbildung mit einer Base der Carboxyfunktionen,
(e) Salzbildung mit einer Säure der Aminofunktionen.

4. Verfahren gemäß Anspruch 1 oder 2, dadurch gekennzeichnet, daß man von einer Verbindung der Formel (II) ausgeht, worin U für einen Rest -CH=CH-CH₂ oder einen Rest -CH₂-CH=CH- steht.

5. Verfahren gemäß einem der Ansprüche 1 bis 4 zur Herstellung von Produkten der Formel (I), worin Y unter den Resten des Alanins, Prolins, Lysins und der α - oder γ-Glutaminsäure sowie deren Estern mit bis zu 6 Kohlenstoffatomen enthaltenden Alkoholen, deren primären und sekundären Amiden und deren Salzen mit organischen oder Mineralsäuren oder mit Basen ausgewählt ist, dadurch gekennzeichnet, daß man auf geeignete Weise die freie Aminfunktion des Produkts der Formel XI oder diejenige des Produkts, erhalten nach Abspaltung der Schutzgruppe der Funktion -NH acyl des Produkts der Formel IV, amidiert.

6. Verfahren gemäß einem der Ansprüche 1 bis 4 zur Herstellung der Produkte der Formel (I), worin Y den Alaninrest bedeutet, sowie von deren Estern mit bis zu 6 Kohlenstoffatome enthaltenden Alkoholen, deren primären und sekundären Amiden und deren Salzen mit organischen oder Mineralsäuren oder mit Basen, dadurch gekennzeichnet, daß man auf geeignete Weise die freie Aminofunktion des Produkts der Formel XI oder diejenige des Produkts, erhalten nach Abspaltung der Schutzgruppe der Funktion -NH acyl des Produkts der Formel IV, amidiert.

7. Verfahren gemäß einem der Ansprüche 1 bis 4 zur Herstellung der Produkte der Formel (I), worin Y den Lysinrest bedeutet, sowie von deren Estern mit bis zu 6 Kohlenstoffatome enthaltenden Alkoholen, deren primären und sekundären Amiden und deren Salzen mit organischen oder Mineralsäuren oder mit Basen, dadurch gekennzeichnet,daß man auf geeignete Weise die freie Aminofunktion des Produkts der Formel XI oder diejenige des Produkts, erhalten nach Abspaltung der Schutzgruppe der Funktion -NH acyl des Produkts der Formel IV, amidiert.

8. Verfahren gemäß einem der Ansprüche 1 bis 3 zur Herstellung der Produkte der Formel (I), worin R für ein Wasserstoffatom steht, sowie von deren Estern mit bis zu 6 Kohlenstoffatome enthaltenden Alkoholen, deren primären und sekundären Amiden und deren Salzen mit organischen oder Mineralsäuren oder mit Basen, dadurch gekennzeichnet, daß man von einem Produkt der Formel II ausgeht, worin X ein Wasserstoffatom bedeutet, oder das Produkt der Formel IV einem geeigneten Decarboxylierungsreagens unterzieht.

9. Verfahren gemäß einem der Ansprüche 1 bis 3 zur Herstellung der Produkte der Formel (I), worin R einen Methylrest bedeutet, sowie von deren Estern mit bis zu 6 Kohlenstoffatome enthaltenden Alkoholen, deren primären und sekundären Amiden und deren Salzen mit organischen oder Mineralsäuren oder mit Basen, dadurch gekennzeichnet, daß man von einem Produkt der Formel II ausgeht, worin X einen Methylrest bedeutet.

10. Verfahren gemäß einem der Ansprüche 1 bis 3 zur Herstellung von Produkten der Formel (I), worin R einen Rest CHF₂ oder auch Ethinyl oder Vinyl bedeutet, sowie von deren Estern mit bis zu 6 Kohlenstoffatome enthaltenden Alkoholen, deren primären und sekundären Amiden und deren Salzen mit organischen oder Mineralsäuren oder mit Basen, dadurch gekennzeichnet, daß man von einem Produkt der Formel II ausgeht, worin X für einen Rest CHF₂ oder auch Ethinyl oder Vinyl steht.

11. Verfahren gemäß Anspruch 1, dadurch gekennzeichnet, daß man das Ausgangsprodukt derart auswählt, daß man die 6-(L-Lysylamino)-3-heptendisäure sowie ihre Ester mit bis zu 6 Kohlenstoffatome enthaltenden Alkoholen, deren primäre und sekundäre Amide und deren Salze mit Basen herstellt.

12. Verfahren gemäß Anspruch 1, dadurch gekennzeichnet, daß man das Ausgangsprodukt derart auswählt, daß man herstellt:
die 6-(L-Alanylamino)-3-heptendisäure,
die 2-(L-Alanylamino)-3-heptendisäure,
sowie deren Ester mit bis zu 6 Kohlenstoffatome enthaltenden Alkoholen, deren primäre und sekundäre Amide und deren Salze mit Basen.
